# EUROPÄISCHE PATENTSCHRIFT

(11) **EP 4 712 843 B1**
(45) Veröffentlichungstag und Bekanntmachung des Hinweises auf die Patenterteilung: **25.11.2026**
(21) Anmeldenummer: 24746045.4
(22) Anmeldetag: 18.05.2024
(51) Int. Cl.: A61B 5/01, A61B 5/00, A61B 5/1455

(54) **TRAGBARE VORRICHTUNG**
PORTABLE DEVICE
DISPOSITIF PORTABLE

(30) Priorität: 18.05.2023 DE 102023113156
(43) Veröffentlichungstag der Anmeldung: 25.03.2026
(73) Patentinhaber: UNIWEARABLES GMBH, 04179 Leipzig (DE)
(72) Erfinder: BLENDINGER, Maximilian Carlos, 10717 Berlin (DE); LANG, Raphael Patrick Manfred, 72631 Aichtal (DE)
(74) Vertreter: Wimmer, Stephan
(86) Internationale Anmeldenummer: PCT/IB2024/054848
(87) Internationale Veröffentlichungsnummer: WO 2024/236548

(56) Entgegenhaltungen:
- US-A1- 2020 221 968
- US-A1- 2021 212 603
- US-A1- 2021 275 095
- US-A1- 2022 128 951
- US-B1- 11 141 129

## Beschreibung

Die vorliegende Erfindung ist auf ein tragbares Datenmodul, ein tragbares Funktionsmodul und eine tragbare Vorrichtung mit einem tragbaren Datenmodul und einem tragbaren Funktionsmodul bezogen, die am Körper eines menschlichen oder tierischen Lebewesens oder medizinischen Personals befestigbar sind, insbesondere zum Erfassen von einem oder mehreren Vitalparametern und/oder zum Erfassen, Verarbeiten, Speichern oder Senden von Daten.

In US 2008/0139953 A1 ist ein am Körper zu tragender physiologischer Sensor beschrieben, der ein wiederverwendbares Kommunikations- und Berechnungsmodul und ein zur Einmalverwendung vorgesehenes Elektrodenmodul umfasst.

In US 2014/0051946 A1 ist eine wiederverwendbare drahtlose Vorrichtung beschrieben, die eine zur Einmalverwendung vorgesehene Komponente mit einer Klebeschicht, Elektroden, elektrischen Kontakten und einer mechanischen Rastverbindungseinrichtung und eine wiederverwendbare Komponente mit einem elektronischen Modul in einem Gehäuse, das mit der Rastverbindungseinrichtung verbindbar ist, umfasst.

In US 2014/0358041 A1 (auch veröffentlicht als WO 2013/158095 A1 und DE 11 2012 005 605 T5) ist das Erfassen der physischen Stabilität eines Patienten mittels eines vom Patienten getragenen Akzelerometers beschrieben. Das Akzelerometer erfasst Vibrationen, aus denen ein Verarbeitungssystem Herzfrequenz und Atemfrequenz bestimmt (Absatz [0009]).

In DE 10 2015 017 430 B3 sind ein Handschuh und ein Armband beschrieben. Der Handschuh ist mit einem Ende eines Verbindungsstücks verbunden. An dem anderen Ende des Verbindungsstücks ist ein Stecker vorgesehen, mit dem das Verbindungsstück in das Armband eingesteckt werden kann.

In WO 2016/010983 A1 ist ein Gesundheitspflaster beschrieben, das eine wiederverwendbare primäre Komponente mit elektronischen Komponenten und eine zur einmaligen Verwendung vorgesehene sekundäre Komponente umfasst. Die primäre Komponente und die sekundäre Komponente können durch Magneten verbunden sein. Das Gesundheitspflaster kann einen Drei-Achsen-Beschleunigungssensor zum Erfassen von Bewegung oder Inaktivität, Temperatursensoren, Elektroden umfassen.

In US 2017/0265769 A1 ist ein Gesundheitsüberwachungs-Pflaster beschrieben, an das ein zur einmaligen Verwendung vorgesehener Elektrodenstreifen geklebt wird.

In US 2019/0150739 A1 (auch veröffentlicht als WO 2019/099643 A1, EP 3 713 485 B1, US 11,389,063 B2) ist ein modularer Vitalparametermonitor beschrieben. Ein Ausrüstungsgehäuse ist durch Rastverbindungen mit zwei Modulen mit jeweils zwei Elektroden verbunden.

In DE 20 2020 103 225 U1 ist ein Vibrations- oder Schallaufnehmer zur Aufnahme von Körper- oder Luftschall an einem Bett angeordnet und mit einer Auswerteeinheit verbunden. Das Erfassen von Vibrationen erlaubt eine Bestimmung von Puls- und Atemfrequenz.

In US 2020/0222003 A1 ist eine auf der Haut tragbare Vorrichtung beschrieben. Eine Tragstruktur umfasst mehrere starre oder im Wesentlichen starre Abschnitte, an denen starre elektronische Komponenten angeordnet sind, und zwischen denen Lücken vorgesehen sind, die nicht-starre Abschnitte bilden.

In US 2021/0177292 A1 ist ein Pflaster zur Überwachung von Vitalparametern beschrieben, das ein Display aufweist.

In US 2021/0212603 A1 ist ein austauschbares Sensorsystem beschrieben. Ein Übertragungsmodul umfasst ein Verarbeitungsmodul und Magneten zur Befestigung eines Sensors an dem Übertragungsmodul.

In US 2022/0128951 A1 ist eine Gesundheitsüberwachungsvorrichtung beschrieben, die durch Magneten mit einer Pflasteranordnung verbindbar ist.

In WO 2022/020874 A1 ist ein System zur Erfassung der Schlafqualität beschrieben. Ein Sensorsystem innerhalb einer Schlafmatratze umfasst einen Beschleunigungssensor, der dazu eingerichtet ist, die durch den Herzschlag und/oder durch die Atmung einer auf der Schlafmatratze liegenden Person erzeugten Beschleunigungssignale zu erfassen (Seite 8, letzter Absatz, Anspruch 1).

In WO 2023/065029 A1 ist ein System zur elektrischen Muskelstimulation beschrieben. Eine Stimulationsvorrichtung weist ein Gehäuse mit einer Vertiefung und Magneten innerhalb des Gehäuses auf. Innerhalb der Vertiefung ist eine Reihe Kontaktstifte angeordnet. Eine Elektrodenvorrichtung weist eine Verbindungseinrichtung mit Magneten und einer Reihe Öffnungen zu Kontaktflächen auf.

Eine Aufgabe der vorliegenden Erfindung besteht darin, ein verbessertes tragbares Datenmodul, ein verbessertes tragbares Funktionsmodul und eine verbesserte tragbare Vorrichtung mit einem Datenmodul und einem Funktionsmodul zu schaffen.

Diese Aufgabe wird durch die Gegenstände der unabhängigen Ansprüche gelöst.

Weiterbildungen sind in den abhängigen Ansprüchen angegeben.

Ausführungsformen der vorliegenden Erfindung beruhen auf der Idee, bei einer modularen tragbaren Vorrichtung ein Datenmodul und ein Funktionsmodul durch die Wechselwirkung von Magneten lösbar mechanisch zu verbinden und an jedem Modul elektrische Kontakte zur elektrischen Verbindung der Module in einer ersten Gruppe und in einer zweiten Gruppe anzuordnen, wobei mindestens ein Magnet an der ersten Gruppe elektrischer Kontakte und mindestens ein Magnet an der zweiten Gruppe elektrischer Kontakte angeordnet ist.

Ein tragbares Datenmodul zur Befestigung am Körper eines menschlichen oder tierischen Lebewesens und zum Erfassen, Verarbeiten, Speichern oder Senden von Daten umfasst eine Schnittstelle zum mechanischen und elektrischen Verbinden des Datenmoduls mit einem Funktionsmodul, wobei die Schnittstelle Magneten zur magnetischen Wechselwirkung mit korrespondierenden Magneten des Funktionsmoduls und elektrische Kontakte zur Bildung lösbarer elektrisch leitfähiger Verbindungen mit korrespondierenden elektrischen Kontakten des Funktionsmoduls umfasst, wobei eine erste Gruppe elektrischer Kontakte und eine zweite Gruppe elektrischer Kontakte von einander beabstandet sind, wobei ein erster Magnet an der ersten Gruppe elektrischer Kontakte angeordnet ist und ein zweiter Magnet an der zweiten Gruppe elektrischer Kontakte angeordnet ist.

Ein tragbares Datenmodul zur Befestigung am Körper eines menschlichen oder tierischen Lebewesens und zum Erfassen, Verarbeiten, Speichern oder Senden von Daten umfasst eine Schnittstelle zum mechanischen und elektrischen Verbinden des Datenmoduls mit einem Funktionsmodul, wobei die Schnittstelle Magneten zur magnetischen Wechselwirkung mit korrespondierenden Magneten des Funktionsmoduls und elektrische Kontakte zur Bildung lösbarer elektrisch leitfähiger Verbindungen mit korrespondierenden elektrischen Kontakten des Funktionsmoduls umfasst, wobei alle elektrischen Kontakte in mehreren Gruppen mit jeweils mehreren elektrischen Kontakten angeordnet sind, wobei alle Abstände zwischen nächst benachbarten elektrischen Kontakten innerhalb einer Gruppe kleiner sind als alle Abstände zwischen je zwei elektrischen Kontakten aus verschiedenen nächst benachbarten Gruppen, wobei ein erster Magnet an einer ersten Gruppe elektrischer Kontakte angeordnet ist und ein zweiter Magnet an einer zweiten Gruppe elektrischer Kontakte angeordnet ist.

Das tragbare Datenmodul kann für eine unmittelbare oder vorzugsweise mittelbare und jedenfalls lösbare mechanische Befestigung an einem Körper einer menschlichen oder tierischen Patientin oder eines menschlichen oder tierischen Patienten oder einer betreuten oder gepflegten Person oder medizinischen Personals vorgesehen und ausgebildet sein. Dazu kann das Datenmodul beispielsweise mit einem Funktionsmodul, das auf die Körperoberfläche geklebt werden kann, verbindbar sein. Alternativ kann das Datenmodul mit einem Gurt oder einem Band über oder unter Kleidung an den Körper gebunden werden. Dazu kann das Datenmodul beispielsweise mit einem Funktionsmodul, das einen Gurt oder ein Band umfasst, lösbar mechanisch verbindbar sein. Alternativ kann das Datenmodul zur dauerhaften oder lösbaren mechanischen Verbindung mit einem Kleidungsstück oder einer Orthese vorgesehen und ausgebildet oder in ein Kleidungsstück oder eine Orthese integriert sein.

Das tragbare Datenmodul ist insbesondere zur Verwendung in einer ärztlichen Praxis, in einem Krankenhaus, in einem Pflegeheim oder in einer anderen Einrichtung zur medizinischen und/oder pflegerischen Versorgung vorgesehen. Das Datenmodul kann dabei gleichzeitig oder nacheinander mehrere verschiedene Funktionen erfüllen.

Das tragbare Datenmodul ist insbesondere vorgesehen und ausgebildet zur unmittelbaren Erfassung von einem oder mehreren Vitalparametern oder anderen Parametern, die die Körpertemperatur, die Atmung, die Herztätigkeit, die Tätigkeit des Gehirns, die Konzentration von Zucker im Blut oder eines anderen Stoffs in einer Körperflüssigkeit oder in Gewebe oder eine andere Eigenschaft oder Funktion des Körpers oder eines Organs charakterisieren. Alternativ oder zusätzlich ist das tragbare Datenmodul vorgesehen und ausgebildet zur mittelbaren Erfassung von einem oder mehreren Vitalparametern, beispielsweise mittels eines Funktionsmoduls, das Elektroden für resisitive oder kapazitive Messungen oder die Messung von Potentialen oder ein Thermometer oder einen oder mehrere optische oder chemische oder andere Sensoren umfasst. Alternativ oder zusätzlich ist das tragbare Datenmodul vorgesehen und ausgebildet zur mechanischen und funktionalen Kopplung mit einem Funktionsmodul, das einen Sensorsignaleingang zum Empfangen eines Sensorsignals von einem Sensor aufweist.

Das tragbare Datenmodul kann alternativ oder zusätzlich vorgesehen und ausgebildet sein, um von medizinischem Personal verwendet und am Körper getragen oder mitgeführt zu werden. Dabei ist das tragbare Datenmodul insbesondere vorgesehen und ausgebildet, um Daten aus anderen, insbesondere baugleichen Datenmodulen auszulesen, medizinische oder pflegerische Tätigkeiten zu dokumentieren, Diagnosen oder Therapieentscheidungen zu erfassen und/oder weiterzuleiten. Dazu kann das Datenmodul unmittelbar oder mittelbar mit dem Körper des medizinischen Personals lösbar verbunden sein, beispielsweise mittels eines Gurts oder eines Bands am Handgelenk.

Das tragbare Datenmodul kann vorgesehen und ausgebildet sein, um zur Erfüllung unterschiedlicher Aufgaben und Funktionen mit unterschiedlichen Funktionsmodulen verbunden zu werden. Das tragbare Datenmodul kann insbesondere vorgesehen und ausgebildet sein, um mit einem ersten Funktionsmodul eine oder mehrere erste Funktionen zu erfüllen und mit einem zweiten Funktionsmodul eine oder mehrere zweite Funktionen zu erfüllen. Beispielsweise ist dasselbe tragbare Datenmodul alternativ mechanisch und elektrisch koppelbar und verwendbar mit einem ersten Funktionsmodul und mit einem zweiten Funktionsmodul. Das erste Funktionsmodul weist beispielsweise eine Klebeschicht und Elektroden auf, um an der Oberfläche des Körpers einer Patientin oder eines Patienten befestigt zu werden, nämlich im Bereich des Brustkorbs auf die Haut geklebt zu werden. Dort kann das Funktionsmodul Vitalparameter der Patientin oder des Patienten erfassen. Das zweite Funktionsmodul weist beispielsweise ein Armband und eine Einrichtung zum Erfassen eines Barcodes oder eines 2D-Codes und/oder einer Benutzerschnittstelle auf. Medizinisches Personal kann das zweite Funktionsmodul mittels des Armbands am Handgelenk befestigen, um Patientendaten zu erfassen, medizinische oder pflegerische Maßnahmen zu dokumentieren, eine Diagnose zu erfassen oder eine therapeutische Maßnahme zu definieren.

Das tragbare Datenmodul ist insbesondere vorgesehen und ausgebildet, um Daten in Form elektrischer oder optischer oder akustischer oder anderer Signale selbst zu generieren oder von einem Funktionsmodul zu empfangen, zu erfassen, zu verarbeiten, zu speichern und/oder zu senden. Alternativ oder zusätzlich kann das tragbare Datenmodul vorgesehen und ausgebildet sein, um Daten von anderen Vorrichtungen, beispielsweise anderen baugleichen tragbaren Datenmodulen zu empfangen. Dazu kann das tragbare Datenmodul beispielsweise eine Funkschnittstelle zum Senden und/oder Empfangen von Daten über eine Funkstrecke oder ein Funknetzwerk umfassen. Die Funkschnittstelle kann einem Industriestandard entsprechen, beispielsweise WLAN (IEEE-802.11), Bluetooth, ZigBee (aufbauend auf IEEE 802.15.4) oder der von der LoRa Alliance festgelegten LoRaWAN-Spezifikation.

Die Magneten der Schnittstelle sind insbesondere zur Erzeugung einer Anziehungskraft mit korrespondierenden Magneten eines Funktionsmoduls vorgesehen und ausgebildet, das heißt vor allem positioniert und orientiert. Von Magneten der Schnittstelle und korrespondierenden Magneten an einem Funktionsmodul erzeugte Anziehungskräfte können eine mechanische, nämlich kraftschlüssige Verbindung zwischen dem tragbaren Datenmodul und dem Funktionsmodul schaffen.

Die Schnittstelle umfasst elektrische Kontakte zum Übertragen und Empfangen elektrischer Leistung und/oder elektrischer Signale, die Steuer- oder Datensignale sein können. Insbesondere sind alle elektrischen Kontakte in mindestens zwei, insbesondere in genau zwei Gruppen mit jeweils mehreren elektrischen Kontakten angeordnet. Dabei sind alle Abstände zwischen elektrischen Kontakten innerhalb einer Gruppe kleiner als der Abstand zwischen den Gruppen. Insbesondere sind alle Abstände zwischen nächst benachbarten elektrischen Kontakten innerhalb einer Gruppe deutlich kleiner als alle Abstände zwischen je zwei elektrischen Kontakten aus verschiedenen Gruppen.

Insbesondere sind alle Abstände zwischen nächst benachbarten elektrischen Kontakten (oder zwischen allen elektrischen Kontakten) innerhalb einer Gruppe höchstens halb so groß wie der Abstand zwischen den Gruppen, also höchstens halb so groß wie der kleinste Abstand zwischen zwei elektrischen Kontakten aus verschiedenen Gruppen.

Die Anordnung der elektrischen Kontakte in zwei oder mehr Gruppen kann mechanische Vorteile schaffen und hinsichtlich der zu bildenden Schaltkreise vorteilhaft sein. Die Anordnung der elektrischen Kontakte in zwei oder mehr Gruppen kann ferner eine störungsfreie oder störungsarme Übertragung von Signalen und/oder eine Codierung von Information - beispielsweise hinsichtlich der Identität eines gekoppelten Funktionsmoduls - in der Anordnung der elektrischen Kontakte ermöglichen.

Die Anordnung je mindestens eines Magneten an jeder von zwei oder mehr Gruppen elektrischer Kontakte kann die Kraft, mit der jeder einzelne elektrische Kontakt gegen einen korrespondierenden elektrischen Kontakt an einem Funktionsmodul gedrückt wird, erhöhen. Dies kann den elektrischen Kontaktwiderstand senken und die Zuverlässigkeit der Kontaktierung verbessern.

Ein tragbares Datenmodul, wie es hier beschrieben ist, weist insbesondere ferner einen konkaven äußeren Oberflächenbereich auf, wobei zumindest entweder elektrische Kontakte der Schnittstelle Teile des konkaven Oberflächenbereichs bilden oder ein Magnet der Schnittstelle unter dem konkaven Oberflächenbereich angeordnet ist.

Der konkave äußere Oberflächenbereich bildet insbesondere eine Vertiefung der äußeren Oberfläche des Datenmoduls, in die ein korrespondierend geformter Bereich eines Funktionsmoduls eingreifen kann.

Der konkave äußere Oberflächenbereich kann den Schutz der elektrischen Kontakte vor mechanischen Einwirkungen verbessern. Ferner kann der konkave äußere Oberflächenbereich eine formschlüssige Orientierung des tragbaren Datenmoduls relativ zu einem Funktionsmodul bereits vor einer wirksamen magnetischen Verbindung des tragbaren Datenmoduls mit dem Funktionsmodul ermöglichen.

Der konkave äußere Oberflächenbereich kann eine längliche, beispielsweise eine näherungsweise streifenförmige oder rechteckige Form aufweisen.

Das tragbare Datenmodul weist insbesondere einen ersten konkaven äußeren Oberflächenbereich und einen zweiten konkaven äußeren Oberflächenbereich auf. Die elektrischen Kontakte der ersten Gruppe bilden Teile des ersten konkaven äußeren Oberflächenbereichs, und der erste Magnet ist unter dem ersten konkaven äußeren Oberflächenbereich angeordnet. Die elektrischen Kontakte der zweiten Gruppe bilden Teile des zweiten konkaven äußeren Oberflächenbereichs, und der zweite Magnet ist unter dem zweiten konkaven äußeren Oberflächenbereich angeordnet.

Ein tragbares Datenmodul, wie es hier beschrieben ist, weist insbesondere ferner einen konvexen äußeren Oberflächenbereich auf, wobei zumindest entweder elektrische Kontakte der Schnittstelle Teile des konvexen Oberflächenbereichs bilden oder ein Magnet der Schnittstelle unter dem konvexen Oberflächenbereich angeordnet ist.

Der konvexe äußere Oberflächenbereich bildet insbesondere eine Ausstülpung oder eine Nase oder einen Erker oder einen Vorsprung der äußeren Oberfläche des Datenmoduls. Der konvexe äußere Oberflächenbereich kann insbesondere in eine korrespondierend geformte Ausnehmung eines Funktionsmoduls eingreifen.

Der konvexe äußere Oberflächenbereich kann eine formschlüssige Orientierung des tragbaren Datenmoduls relativ zu einem Funktionsmodul bereits vor einer wirksamen magnetischen Verbindung des tragbaren Datenmoduls mit dem Funktionsmodul ermöglichen.

Der konvexe äußere Oberflächenbereich kann eine längliche, beispielsweise eine näherungsweise streifenförmige oder rechteckige Form aufweisen.

Das tragbare Datenmodul weist insbesondere einen ersten konvexen äußeren Oberflächenbereich und einen zweiten konvexen äußeren Oberflächenbereich auf. Die elektrischen Kontakte der ersten Gruppe bilden Teile des ersten konvexen äußeren Oberflächenbereichs, und der erste Magnet ist unter dem ersten konvexen äußeren Oberflächenbereich angeordnet. Die elektrischen Kontakte der zweiten Gruppe bilden Teile des zweiten konvexen äußeren Oberflächenbereichs, und der zweite Magnet ist unter dem zweiten konvexen äußeren Oberflächenbereich angeordnet.

Ein tragbares Datenmodul, wie es hier beschrieben ist, umfasst insbesondere ferner einen konvexen äußeren Oberflächenbereich an derselben Seite des Datenmoduls, an der auch die elektrischen Kontakte angeordnet sind, zum Eingreifen in eine Ausnehmung des Funktionsmoduls, und einen Sensor an dem konvexen äußeren Oberflächenbereich.

Ein tragbares Datenmodul, wie es hier beschrieben ist, umfasst insbesondere ferner einen konvexen äußeren Oberflächenbereich an derselben Seite des Datenmoduls, an der auch die elektrischen Kontakte angeordnet sind, zum Eingreifen in eine Ausnehmung des Funktionsmoduls, wobei der konvexe Oberflächenbereich keine 2-zählige Rotationssymmetrie aufweist.

Ein tragbares Datenmodul, wie es hier beschrieben ist, umfasst insbesondere ferner einen konvexen äußeren Oberflächenbereich an derselben Seite des Datenmoduls, an der auch die elektrischen Kontakte angeordnet sind, zum Eingreifen in eine Ausnehmung des Funktionsmoduls, und einen Sensor an dem konvexen Oberflächenbereich, wobei der konvexe Oberflächenbereich keine 2-zählige Rotationssymmetrie aufweist.

Der konvexe äußere Oberflächenbereich ist insbesondere als Vorsprung oder Erker oder Nase ausgebildet. Der konvexe äußere Oberflächenbereich ragt insbesondere aus einem im Übrigen weitgehend oder vollständig ebenen Oberflächenbereich heraus.

Der Sensor kann unter dem konvexen Oberflächenbereich angeordnet sein, beispielsweise hinter einem optisch transparenten Fensterbauteil. Alternativ kann eine Oberfläche des Sensors selbst einen Teil des konvexen Oberflächenbereichs bilden.

Der Sensor ist beispielsweise ein optischer Sensor mit einer Lichtquelle zum Erzeugen von Beleuchtungslicht im infraroten Wellenlängenbereich, im für das gesunde menschliche Auge sichtbaren Wellenlängenbereich und/oder im ultravioletten Wellenlängenbereich und einen Lichtsensor. Der Lichtsensor ist insbesondere vorgesehen und ausgebildet zum Erfassen von remittiertem oder reflektiertem Beleuchtungslicht oder von Fluoreszenzlicht, das bei der Beleuchtung von Gewebe mit dem Beleuchtungslicht entsteht. Die Intensität des von dem Lichtsensor erfassten Lichts kann eine Charakterisierung des Gewebes oder einer Sauerstoffkonzentration oder einer anderen Stoffkonzentration in dem Gewebe ermöglichen. Eine zeitliche Modulation des von dem Lichtsensor erfassten Lichts kann weitere Information über das Gewebe oder über physiologische Vorgänge enthalten.

Alternativ oder zusätzlich können an dem konvexen äußeren Oberflächenbereich beispielsweise eine oder mehrere Elektroden vorgesehen sein. Die Elektrode oder die Elektroden können eine kapazitive oder resistive Erfassung eines Parameters, der Gewebe charakterisiert, oder eines elektrischen Potentials, dessen zeitliche Modulation einen physiologischen Vorgang charakterisiert, ermöglichen.

Der konvexe äußere Oberflächenbereich kann ein unmittelbares Anliegen des Sensors, beispielsweise einer Lichtaustrittsfläche und/oder einer Lichteintrittsfläche des Sensors, an der Haut eines Patienten ermöglichen. Der konvexe äußere Oberflächenbereich kann das unmittelbare Anliegen oder einen nur geringen Abstand auch dann ermöglichen, wenn zwischen dem tragbaren Datenmodul und der Haut des Patienten ein Funktionsmodul angeordnet ist. Dazu ragt der konvexe äußere Oberflächenbereich beispielsweise durch eine Durchgangsöffnung in dem Funktionsmodul hindurch.

Der Rand oder die Flanke des konvexen äußeren Oberflächenbereichs ist insbesondere teilweise oder vollständig böschungsartig geneigt.

Eine böschungsartige Ausgestaltung des Rands oder der Flanke des konvexen äußeren Oberflächenbereichs kann eine Ausrichtung des tragbaren Datenmoduls relativ zu dem Funktionsmodul mit einer korrespondierenden Ausnehmung schon bei der Annäherung ermöglichen.

Der konvexe äußere Oberflächenbereich weist beispielsweise einen im Wesentlichen dreieckigen, rechteckigen, trapezförmigen oder anderen polygonalen Grundriss auf. Der Grundriss des konvexen äußeren Oberflächenbereichs kann punktsymmetrisch oder spiegelsymmetrisch zu einer Symmetrieebene sein oder eine andere Symmetrie aufweisen oder keine Symmetrie aufweisen.

Eine asymmetrische Ausbildung kann eine eindeutige Codierung einer einzigen möglichen Orientierung des tragbaren Datenmoduls relativ zu dem Funktionsmodul mit korrespondierender Ausnehmung ermöglichen. Eine Ausgestaltung des konvexen Oberflächenbereichs ohne eine 2-zählige Rotationssymmetrie kann zusammen mit weiteren Merkmalen, beispielsweise der Anordnung der Schnittstelle eine einzige mögliche Orientierung des tragbaren Datenmoduls relativ zu dem Funktionsmodul eindeutig codieren.

Ein tragbares Datenmodul, wie es hier beschrieben ist, umfasst insbesondere ferner ein Gehäuse, das die äußere Oberfläche des Datenmoduls bildet, wobei das Gehäuse ein erstes Gehäuseteil und ein zweites Gehäuseteil und eine Dichteinrichtung aus einem elastischen Material zwischen dem ersten Gehäuseteil und dem zweiten Gehäuseteil aufweist, wobei die Dichteinrichtung zumindest teilweise zwischen einem Steg an einem Rand des ersten Gehäuseteils, der einen Teil der äußeren Oberfläche des Datenmoduls bildet, und einem Steg an einem Rand des zweiten Gehäuseteils, der einen Teil einer inneren Oberfläche des Gehäuses bildet, gequetscht ist.

Diese Anordnung der Dichteinrichtung bewirkt beim Schließen des Gehäuses, also der gegenseitigen Annäherung der Gehäuseteile eine Scherkraft auf die Dichteinrichtung, die durch gleitfähige korrespondierende Oberflächen an der Dichteinrichtung und an mindestens einem der Gehäuseteile reduziert werden kann. Vor allem aber ist die Normalkraft zwischen den korrespondierenden Oberflächen der Dichteinrichtung und der Gehäuseteile erhöht. Dies kann die Dichtwirkung verbessern. Außerdem können die korrespondierenden Dichtflächen an Dichteinrichtung und Gehäuseteilen vergrößert sein, was ebenfalls der Dichtwirkung und ihrer Dauerhaftigkeit zugute kommen kann.

Ein tragbares Datenmodul, wie es hier beschrieben ist, umfasst insbesondere ferner ein Gehäuse, das die äußere Oberfläche des tragbaren Datenmoduls bildet, und einen Betätigungssensor, wobei das Gehäuse ein erstes Gehäuseteil und ein zweites Gehäuseteil und eine Dichteinrichtung aus einem elastischen Material zwischen dem ersten Gehäuseteil und dem zweiten Gehäuseteil aufweist, wobei die Dichteinrichtung einen elastischen Oberflächenbereich der äußeren Oberfläche des tragbaren Datenmoduls bildet, dessen manuelle Verformung durch den Betätigungssensor erfasst wird.

Der elastische Oberflächenbereich weist aufgrund der Elastizität des Materials der Dichteinrichtung und aufgrund seiner Geometrie eine größere Elastizität als andere Bereiche der äußeren Oberfläche auf. Insbesondere wird der elastische Oberflächenbereich durch eine lokal stark verbreiterte Dichtfuge gebildet.

Der Betätigungssensor kann einen Schalter oder eine andere Einrichtung zum Erfassen einer Verformung des elastischen Oberflächenbereichs umfassen. Da der Betätigungssensor insbesondere innerhalb des Gehäuses angeordnet ist, erfasst er die Verformung des elastischen äußeren Oberflächenbereichs insbesondere mittelbar, indem er die Verformung eines gegenüberliegenden inneren Oberflächenbereichs der Dichteinrichtung erfasst.

Die Anordnung aus einem Betätigungssensor innerhalb des Gehäuses und einem durch die Dichteinrichtung gebildeten und manuell verformbaren elastischen äußeren Oberflächenbereich schafft eine Benutzerschnittstelle ohne die Komplexität der äußeren Oberfläche des tragbaren Datenmoduls zu erhöhen. Insbesondere ist weder ein zusätzliches Bauteil noch eine zusätzliche Dichtfläche erforderlich. Dies kann eine hermetisch dichte Ausgestaltung des Gehäuses bei günstigen Fertigungskosten vereinfachen.

Bei einem tragbaren Datenmodul, wie es hier beschrieben ist, ist das Gehäuse insbesondere aus einem transparenten Material gebildet, wobei innerhalb des Gehäuses erzeugtes oder gestreutes oder reflektiertes Licht durch das Gehäuse austreten kann, um außerhalb des Gehäuses wahrgenommen zu werden.

Das transparente Material des Gehäuses ist insbesondere für für das gesunde menschliche Auge sichtbares Licht transparent. Alternativ oder zusätzlich ist das transparente Material für infrarotes und/oder ultraviolettes Licht transparent.

Durch die Ausbildung des Gehäuses aus einem transparenten Material kann Licht einer Leuchtdiode oder von einem selbst leuchtenden Display erzeugtes Licht oder an einem Display ohne Hintergrundbeleuchtung reflektiertes oder remittiertes Licht aus dem Gehäuse austreten, um außerhalb des Gehäuses unmittelbar mit den Augen wahrgenommen oder mittels einer Kamera oder einer anderen Einrichtung erfasst zu werden.

Optional kann die innere und/oder die äußere Oberfläche des Gehäuses in einem oder mehreren Bereichen oder vollständig rau ausgebildet sein. Dadurch können diese Bereiche oder das gesamte Gehäuse opak wirken. Dies ermöglicht das Verbergen von Einrichtungen im Inneren des Gehäuses. Intensität und Wellenlänge oder Farbe von Licht einer Lichtquelle innerhalb des Gehäuses können jedoch auch durch einen opaken Bereich des Gehäuses hindurch erkennbar sein. Dies kann ermöglicht sowohl die Übermittlung von Information an eine Person, die das tragbare Datenmodul verwendet oder betrachtet, als auch den Austausch von Information zwischen dem tragbaren Datenmodul und einer anderen Vorrichtung in beiden Richtungen ermöglichen. Eine raue äußere Oberfläche kann ferner die Erkennbarkeit einer Verschmutzung der äußeren Oberfläche verbessern und die Haptik beeinflussen.

Alternativ kann das Gehäuse teilweise oder vollständig aus einem opaken Material gebildet sein.

Ein tragbares Datenmodul, wie es hier beschrieben ist, umfasst insbesondere ferner einen Beschleunigungssensor zum Erfassen einer Beschleunigung des Datenmoduls und zum Erzeugen eines Sensorsignals, das die erfasste Beschleunigung repräsentiert, und eine Auswerteeinrichtung zum Empfangen und Auswerten des Sensorsignals und zum Bestimmen zumindest eines Parameters, der die Herzaktion oder die Atemtätigkeit oder die körperliche Aktivität oder die Position und Orientierung im Raum charakterisiert, aus dem Sensorsignal.

Der Beschleunigungssensor erfasst insbesondere den Herzschlag und/oder die Atmung, um beispielsweise die Herzfrequenz oder den Herzrhythmus oder die Atemfrequenz zu bestimmen.

Ein tragbares Datenmodul, wie es hier beschrieben ist, umfasst insbesondere ferner eine Anzeigeeinrichtung zum visuell erfassbaren optischen Anzeigen von Information, wobei die Anzeigeeinrichtung ausgebildet ist, um auch ohne Leistungsversorgung Information darzustellen.

Die Anzeigeeinrichtung umfasst insbesondere eine Technologie, die als elektronische Tinte oder elektronisches Papier bekannt ist, und bei der ein einmaliges Anlegen eines elektrischen Felds zwischen Elektroden beispielsweise eine dauerhafte, aber reversible lokale Umlagerung von Partikeln bewirkt, die die Streuung von Licht beeinflussen.

Bei einem tragbaren Datenmodul, wie es hier beschrieben ist, ist das tragbare Datenmodul insbesondere mit mehreren unterschiedlichen Funktionsmodulen mechanisch und elektrisch verbindbar.

Durch unterschiedliche Funktionsmodule kann das tragbare Datenmodul für unterschiedliche Aufgaben und Funktionen angepasst werden.

Ein tragbares Funktionsmodul zur Befestigung am Körper eines menschlichen oder tierischen Lebewesens und zum Erfassen eines Messwerts umfasst eine Schnittstelle zum mechanischen und elektrischen Verbinden des Funktionsmoduls mit einem tragbaren Datenmodul, wobei die Schnittstelle Magneten zur magnetischen Wechselwirkung mit korrespondierenden Magneten des Datenmoduls und elektrische Kontakte zur Bildung lösbarer elektrisch leitfähiger Verbindungen mit korrespondierenden elektrischen Kontakten des Datenmoduls umfasst, wobei eine erste Gruppe elektrischer Kontakte und eine zweite Gruppe elektrischer Kontakte von einander beanstandet sind, wobei ein erster Magnet an der ersten Gruppe elektrischer Kontakte angeordnet ist und ein zweiter Magnet an der zweiten Gruppe elektrischer Kontakte angeordnet ist.

Ein tragbares Funktionsmodul zur Befestigung am Körper eines menschlichen oder tierischen Lebewesens und zum Erfassen eines Messwerts umfasst eine Schnittstelle zum mechanischen und elektrischen Verbinden des Funktionsmoduls mit einem tragbaren Datenmodul, wobei die Schnittstelle Magneten zur magnetischen Wechselwirkung mit korrespondierenden Magneten des Datenmoduls und elektrische Kontakte zur Bildung lösbarer elektrisch leitfähiger Verbindungen mit korrespondierenden elektrischen Kontakten des Datenmoduls umfasst, wobei alle elektrischen Kontakte in mehreren Gruppen mit jeweils mehreren elektrischen Kontakten angeordnet sind, wobei alle Abstände zwischen nächst benachbarten elektrischen Kontakten innerhalb einer Gruppe kleiner sind als alle Abstände zwischen je zwei elektrischen Kontakten aus verschiedenen nächst benachbarten Gruppen, wobei ein erster Magnet an der ersten Gruppe elektrischer Kontakte angeordnet ist und ein zweiter Magnet an der zweiten Gruppe elektrischer Kontakte angeordnet ist.

Die Schnittstelle des tragbaren Funktionsmoduls ist insbesondere vorgesehen und ausgebildet zum mechanischen und elektrischen Verbinden des tragbaren Funktionsmoduls mit einer korrespondierenden Schnittstelle eines tragbaren Datenmoduls, wie es hier beschrieben ist. Die Magneten der Schnittstelle sind insbesondere zur Erzeugung einer Anziehungskraft mit korrespondierenden Magneten eines tragbaren Datenmoduls vorgesehen und ausgebildet, das heißt vor allem positioniert und orientiert. Von Magneten der Schnittstelle des tragbaren Funktionsmoduls und korrespondierenden Magneten an einem tragbaren Datenmodul erzeugte Anziehungskräfte können eine mechanische, nämlich kraftschlüssige Verbindung zwischen dem tragbaren Funktionsmodul und dem tragbaren Datenmodul schaffen.

Das tragbare Funktionsmodul kann die mittelbare Befestigung des Datenmoduls am Körper einer menschlichen oder tierischen Patientin oder eines menschlichen oder tierischen Patienten oder am Körper medizinischen Personals ermöglichen. Dazu weist das tragbare Funktionsmodul beispielsweise eine Klebeschicht auf, mittels derer das Funktionsmodul auf die Haut oder auf ein Kleidungsstück geklebt werden kann. Alternativ oder zusätzlich kann das tragbare Funktionsmodul ein Band oder einen Gurt oder eine Befestigungseinrichtung für ein Band oder ein Gurt aufweisen, um mittels des Bands oder Gurts am Handgelenk, am Unterarm, am Oberarm, an einer anderen Extremität, am Oberkörper oder am Kopf befestigt zu werden. Alternativ oder zusätzlich kann das tragbare Funktionsmodul vorgesehen und ausgebildet sein, um mit einer Orthese oder einem Kleidungsstück mechanisch verbunden zu werden oder kann selbst als Orthese oder als Kleidungsstück ausgebildet sein.

Das tragbare Funktionsmodul kann eine oder mehrere Elektroden aufweisen, um resisitive oder kapazitive Messungen zu ermöglichen oder das elektrische Potential an der Körperoberfläche zu erfassen, beispielsweise für eine Elektrokardiographie oder eine Elektroenzephalographie. Alternativ oder zusätzlich kann das Funktionsmodul einen oder mehrere optische oder andere Sensoren zur Erfassung von einem oder mehreren Vitalparametern oder anderen Parametern, die die Körpertemperatur, die Atmung, die Herztätigkeit, die Tätigkeit des Gehirns, die Konzentration von Zucker im Blut oder eines anderen Stoffs in einer Körperflüssigkeit oder in Gewebe oder eine andere Körperfunktion charakterisieren, aufweisen.

Alternativ oder zusätzlich kann das Funktionsmodul eine Kamera oder einen Scanner zum optischen Erfassen eines ein- oder zweidimensionalen Codes aufweisen.

Die Schnittstelle umfasst elektrische Kontakte zum Übertragen und Empfangen elektrischer Leistung und/oder elektrischer Signale, die Steuer- oder Datensignale sein können. Insbesondere sind alle elektrischen Kontakte in mindestens zwei, insbesondere in genau zwei Gruppen mit jeweils mehreren elektrischen Kontakten angeordnet. Dabei sind alle Abstände zwischen elektrischen Kontakten innerhalb einer Gruppe kleiner als der Abstand zwischen den Gruppen. Insbesondere sind alle Abstände zwischen nächst benachbarten elektrischen Kontakten innerhalb eienr Gruppe deutlich kleiner als alle Abstände zwischen je zwei elektrischen Kontakten aus verschiedenen Gruppen.

Insbesondere sind alle Abstände zwischen nächst benachbarten elektrischen Kontakten (oder zwischen allen elektrischen Kontakten) innerhalb einer Gruppe höchstens halb so groß wie der Abstand zwischen den Gruppen, also höchstens halb so groß wie der kleinste Abstand zwischen zwei elektrischen Kontakten aus verschiedenen Gruppen.

Die Anordnung der elektrischen Kontakte in zwei oder mehr Gruppen kann mechanische Vorteile schaffen und hinsichtlich der zu bildenden Schaltkreise vorteilhaft sein. Die Anordnung der elektrischen Kontakte in zwei oder mehr Gruppen kann ferner eine störungsfreie oder störungsarme Übertragung von Signalen und/oder eine Codierung von Information - beispielsweise hinsichtlich der Identität eines gekoppelten Funktionsmoduls - in der Anordnung der elektrischen Kontakte ermöglichen.

Die Anordnung je mindestens eines Magneten an jeder von zwei oder mehr Gruppen elektrischer Kontakte kann die Kraft, mit der jeder einzelne elektrische Kontakt gegen einen korrespondierenden elektrischen Kontakt an einem tragbaren Datenmodul gedrückt wird, erhöhen. Dies kann den elektrischen Kontaktwiderstand senken und die Zuverlässigkeit der Kontaktierung verbessern.

Ein tragbares Funktionsmodul, wie es hier beschrieben ist, umfasst insbesondere ferner einen konvexen äußeren Oberflächenbereich, wobei zumindest entweder die elektrischen Kontakte Teile des konvexen Oberflächenbereichs bilden oder die Magneten unter dem konvexen Oberflächenbereich angeordnet sind.

Der konvexe äußere Oberflächenbereich ist insbesondere als Vorsprung oder Erhebung oder Erker oder Nase ausgebildet. Der konvexe äußere Oberflächenbereich ragt insbesondere aus einem im Übrigen weitgehend oder vollständig ebenen Oberflächenbereich heraus.

Der konvexe Oberflächenbereich kann eine formschlüssige Orientierung des tragbaren Funktionsmoduls relativ zu einem tragbaren Datenmodul bereits vor einer wirksamen magnetischen Verbindung des tragbaren Funktionsmoduls mit dem tragbaren Datenmodul ermöglichen.

Das tragbare Funktionsmodul weist insbesondere einen ersten konvexen äußeren Oberflächenbereich und einen zweiten konvexen äußeren Oberflächenbereich auf. Die elektrischen Kontakte der ersten Gruppe bilden Teile des ersten konvexen äußeren Oberflächenbereichs, und der erste Magnet ist unter dem ersten konvexen äußeren Oberflächenbereich angeordnet. Die elektrischen Kontakte der zweiten Gruppe bilden Teile des zweiten konvexen äußeren Oberflächenbereichs, und der zweite Magnet ist unter dem zweiten konvexen äußeren Oberflächenbereich angeordnet.

Ein tragbares Funktionsmodul, wie es hier beschrieben ist, umfasst insbesondere ferner einen konkaven äußeren Oberflächenbereich an derselben Seite des Funktionsmoduls, an der auch die elektrischen Kontakte angeordnet sind, zum Aufnehmen eines konvexen Bereichs des Datenmoduls.

Ein tragbares Funktionsmodul, wie es hier beschrieben ist, umfasst insbesondere ferner einen konkaven äußeren Oberflächenbereich an derselben Seite des Funktionsmoduls, an der auch die elektrischen Kontakte angeordnet sind, zum Aufnehmen eines konvexen Bereichs des Datenmoduls, wobei der konkave Oberflächenbereich keine 2-zählige Rotationssymmetrie aufweist.

Der konkave Oberflächenbereich ist insbesondere vorgesehen und ausgebildet, um einen korrespondierenden konvexen Oberflächenbereich eines tragbaren Datenmoduls aufzunehmen. Der konkave Oberflächenbereich kann in Wechselwirkung mit einem korrespondierenden konvexen Oberflächenbereich eines tragbaren Datenmoduls eine formschlüssige Festlegung einer einzigen möglichen Anordnung und Orientierung des tragbaren Funktionsmoduls relativ zu dem tragbaren Datenmodul ermöglichen.

Bei einem tragbaren Funktionsmodul, wie es hier beschrieben ist, ist der konkave äußere Oberflächenbereich insbesondere als Durchgangsöffnung ausgebildet, durch die ein konvexer äußerer Oberflächenbereich eines Datenmoduls hindurchgreifen kann.

Ein tragbares Funktionsmodul, wie es hier beschrieben ist, umfasst insbesondere ferner eine Tragstruktur, mit der die Magneten und die elektrischen Kontakte mechanisch starr verbunden sind, und eine Klebeschicht an einer von den elektrischen Kontakten abgewandten Seite der Tragstruktur, wobei die Tragstruktur eine strukturierte Schicht umfasst, wobei die strukturierte Schicht der Tragstruktur eine kammartige Struktur umfasst.

Eine kammartige Struktur umfasst mehrere parallele gerade oder gekrümmte Zinken oder Finger, die parallel oder im Wesentlichen parallel angeordnet sind.

Eine kammartige Struktur kann eine definierte Elastizität oder Verformbarkeit aufweisen, die in einer Richtung wesentlich größer sein kann als in einer zweiten, orthogonale Richtung. Eine kammartige Struktur kann dämpfend wirken, vor allem auch in Zusammenwirken mit anderen Schichten der Tragstruktur. Dadurch kann eine kammartige Struktur das durch Bewegungen der Patientin oder des Patienten oder des medizinischen Personals angeregte Schaukeln, Wackeln oder Vibrieren des tragbaren Funktionsmoduls reduzieren.

Bei einem tragbaren Funktionsmodul, wie es hier beschrieben ist, umfasst die kammartige Struktur insbesondere mehrere parallele oder im Wesentlichen parallele bogenförmige finger- oder streifenförmige Strukturen.

Eine finger- oder streifenförmige Strukturen ist eine längliche Struktur, deren Länge ein Mehrfaches, insbesondere mindestens ein Dreifaches, ein Fünffaches oder ein Zehnfaches ihrer Breite beträgt. Finger- oder streifenförmige Strukturen sind im Wesentlichen parallel zu einander, wenn ihr Abstand entlang ihrer Längsrichtung nicht oder nur geringfügig variiert, so dass beispielsweise der maximale Abstand nicht mehr als das Doppelte des minimalen Abstands beträgt.

Die finger- oder streifenförmigen Strukturen können jeweils gerade oder gekrümmt, insbesondere kreisbogenförmig gekrümmt sein.

Bei einem tragbaren Funktionsmodul, wie es hier beschrieben ist, umfasst die strukturierte Schicht insbesondere mehrere kammartige Strukturen mit jeweils mehreren parallelen oder im Wesentlichen parallelen bogenförmigen finger- oder streifenförmige Strukturen.

Bei einem tragbaren Funktionsmodul, wie es hier beschrieben ist, sind die kammartigen Strukturen (124) insbesondere spiegelsymmetrisch angeordnet sind.

Die Anordnung der kammartigen Strukturen weist insbesondere zwei Symmetrieebenen auf.

Bei einem tragbaren Funktionsmodul, wie es hier beschrieben ist, gehen die kammartigen Strukturen insbesondere von einem streifenförmigen oder im Wesentlichen streifenförmigen oder im Wesentlichen kreuzförmigen oder knochenförmigen Zentralbereich der strukturierten Schicht aus, wobei die Gruppen elektrischer Kontakte an von einander abgewandten Enden des Zentralbereichs der strukturierten Schicht befestigt sind.

Insbesondere sind auch die Magneten an von einander abgewandten Enden des Zentralbereichs der strukturierten Schicht befestigt.

Der Zentralbereich der strukturierten Schicht weist insbesondere eine geringe Elastizität oder Flexibilität auf und ermöglicht eine weitgehend starre und damit zuverlässige mechanische und elektrische Verbindung des tragbaren Funktionsmoduls mit einem tragbaren Datenmodul.

Bei einem tragbaren Funktionsmodul, wie es hier beschrieben ist, umfasst die Tragstruktur insbesondere ferner eine zumindest weitgehend unstrukturierte Schicht zwischen der strukturierten Schicht und der Klebeschicht, wobei die weitgehend unstrukturierte Schicht flexibler ist als das unstrukturierte Ausgangsmaterial der strukturierten Schicht.

Die zumindest weitgehend unstrukturierte Schicht ist insbesondere vollständig unstrukturiert. Die zumindest weitgehend unstrukturierte Schicht ist insbesondere dünner als das Ausgangsmaterial, aus dem die strukturierte Schicht durch Strukturierung geschaffen ist.

Bei einem tragbaren Funktionsmodul, wie es hier beschrieben ist, ist das Funktionsmodul insbesondere länglich und tailliert.

Insbesondere ist die weitgehend oder vollständig unstrukturierte Schicht länglich und tailliert, also im weiteren Sinne knochenförmig.

Eine taillierte Ausgestaltung des Funktionsmoduls, insbesondere der weitgehend oder vollständig unstrukturierten Schicht kann eine in einer Richtung erhöhte, also anisotrope Flexibilität des Funktionsmoduls bewirken. Die anisotrope Flexibilität des Funktionsmoduls kann gleichzeitig einerseits eine zuverlässige mechanische und elektrische Verbindung mit einem tragbaren Datenmodul und andererseits eine Anpassung an einen gewölbten Oberflächenbereich eines Körpers einer Patientin oder eines Patienten ermöglichen.

Bei einem tragbaren Funktionsmodul, wie es hier beschrieben ist, ist der streifenförmige oder im Wesentlichen streifenförmige Zentralbereich der strukturierten Schicht insbesondere quer zu einer Längsrichtung des tragbaren Funktionsmoduls an oder nahe dessen schmalster Stelle angeordnet ist.

Der Zentralbereich kann also das tragbare Funktionsmodul lokal versteifen und insbesondere die Flexibilität in Richtung quer zur Längsrichtung des tragbaren Funktionsmoduls deutlich reduzieren. Im Übrigen kann die Flexibilität des tragbaren Funktionsmoduls hoch sein und so eine Anpassung an die Krümmung der Oberfläche des Körpers einer Patientin oder eines Patienten ermöglichen.

Ein tragbares Funktionsmodul, wie es hier beschrieben ist, umfasst insbesondere ferner eine Kamera oder einen Scanner zum Erfassen codierter Information.

Das tragbare Funktionsmodul kann insbesondere vorgesehen sein, um in der Hand gehalten und so verwendet zu werden.

Ein tragbares Funktionsmodul, wie es hier beschrieben ist, umfasst insbesondere ferner eine Kamera oder einen Scanner zum Erfassen codierter Information, eine Befestigungseinrichtung zum lösbaren mechanischen Befestigen des tragbaren Funktionsmoduls an einer Hand oder einem Handgelenk oder einem Unterarm oder einem Oberarm oder einem Bein oder einem Oberkörper oder einem Kopf einer Person oder an einem Tier oder an einer weiteren Vorrichtung.

Die Kamera oder der Scanner ist insbesondere zum Erfassen eines (eindimensionalen) Barcodes oder eines QR-Codes oder eines anderen 2D-Codes vorgesehen und ausgebildet.

Die Kamera oder der Scanner kann beispielsweise eine Identifizierung einer Patientin oder eines Patienten ermöglichen, indem ein Code am Bett oder an einem Armband der Patientin oder des Patienten erfasst wird. Alternativ oder zusätzlich kann die Kamera oder der Scanner beispielsweise die Identifizierung eines Medikaments oder einer Therapieeinrichtung ermöglichen und damit die Dokumentation einer Medikation oder einer Therapie vereinfachen.

Ein tragbares Funktionsmodul, wie es hier beschrieben ist, umfasst insbesondere ferner einen Feuchtesensor oder eine Schnittstelle zur Kopplung mit einem Feuchtesensor.

Der Feuchtesensor ist in Klinik oder Pflege insbesondere zur Überwachung einer Wunde oder einer Windel verwendbar.

Ein tragbares Funktionsmodul, wie es hier beschrieben ist, umfasst insbesondere ferner eine Schicht mit einem im Wesentlichen C-förmigen Schlitz, der einen halbinselförmigen Bereich, der nur über einen Steg mit umliegenden Bereichen der Schicht verbunden ist, umschließt, und einen Temperatursensor an dem halbinselförmigen Bereich.

Der Steg ist zwischen den Enden des C-förmigen Schlitzes angeordnet.

Ein tragbares Funktionsmodul, wie es hier beschrieben ist, umfasst insbesondere ferner eine Schicht mit zwei Schlitzen, die einen stegförmigen Bereich abgrenzen, und einen Temperatursensor an dem stegförmigen Bereich.

Die zwei Schlitze sind insbesondere gerade und parallel oder im Wesentlichen parallel, schließen also einen Winkel von nicht mehr als 30 Grad oder nicht mehr als 20 Grad oder nicht mehr als 10 Grad ein. In diesem Fall weist der stegförmige Bereich die Gestalt eines Rechtecks oder eines stumpfen Keils auf.

Alternativ können ein Schlitz oder beide Schlitze polygonal oder gebogen sein. In diesem Fall ist der stegförmige Bereich beispielsweise in einem mittleren Bereich breiter als an seinen Enden oder umgekehrt.

Ein tragbares Funktionsmodul, wie es hier beschrieben ist, umfasst insbesondere ferner eine Schicht mit einer Mehrzahl von Schlitzen, die einen vorbestimmten Bereich teilweise umschließen oder umgeben, und einen Temperatursensor an dem vorbestimmten Bereich.

Die Mehrzahl von Schlitzen können beispielsweise Abschnitte eines Kreisbogen oder eines Rands eines Quadrats oder eines Rechtecks oder eines anderen Polygons sein. Der vorbestimmte Bereich ist nur durch Stege zwischen den Schlitzen mit umgebenden Bereichen der Schicht verbunden.

Ein tragbares Funktionsmodul, wie es hier beschrieben ist, umfasst insbesondere ferner eine Schicht mit einer Mehrzahl von Ausnehmungen, die einen vorbestimmten Bereich teilweise umschließen, und einen Temperatursensor an dem vorbestimmten Bereich.

Der vorbestimmte Bereich ist nur durch Stege zwischen den Ausnehmungen mit umgebenden Bereichen der Schicht verbunden.

Die Schlitze oder Ausnehmungen durchdringen die Schicht insbesondere vollständig. Sie reduzieren die Wärmeleitung in der Schicht und können damit eine empfindlichere, genauere und flinkere oder weniger träge Erfassung einer Temperatur ermöglichen.

Ein tragbares Funktionsmodul, wie es hier beschrieben ist, umfasst insbesondere ferner einen weiteren Temperatursensor an dem halbinselförmigen Bereich oder an dem stegförmigen Bereich oder an dem vorbestimmten Bereich.

Insbesondere sind der Temperatursensor und der weitere Temperatursensor an von einander abgewandten Seiten des halbinselförmigen Bereichs angeordnet, beispielsweise spiegelsymmetrisch zu einer Spiegelebene, die zumindest in dem halbinselförmigen Bereich innerhalb der Schicht liegt.

Die Schicht kann Leiterbahnen oder andere elektrische Leitungen enthalten und dazu insbesondere durch eine starre oder flexible Leiterplatte gebildet sein.

Ein tragbares Funktionsmodul, wie es hier beschrieben ist, umfasst insbesondere ferner einen Drucksensor zum Erfassen eines Drucks oder einer Druckänderung in einem Fluid und eine Fluidleitung mit einem ersten Ende, das mit dem Drucksensor fluidisch gekoppelt ist, wobei der Druck in der Fluidleitung manuell beeinflussbar und durch den Drucksensor erfassbar ist.

Wenn die Fluidleitung elastisch ausgebildet ist, kann der Druck in der Fluidleitung durch unmittelbare oder mittelbare mechanische Kraftausübung auf und Verformung der Fluidleitung verändert werden. In diesem Fall ist das zweite Ende der Fluidleitung insbesondere fluiddicht verschlossen.

Alternativ oder zusätzlich kann beispielsweise ein Balg mit dem zweiten Ende der Fluidleitung verbunden sein. Eine unmittelbar oder mittelbar auf den Balg ausgeübte Kraft kann den Balg verformen und den Fluiddruck in dem Balg und in der Fluidleitung verändern.

Die Fluidleitung oder der Balg sind beispielsweise in ein Armband integriert, so dass durch Bewegung des Handgelenks oder Anspannen der Muskulatur am Unterarm der Fluiddruck veränderbar ist. Die Anordnung aus Drucksensor, Fluidleitung und optional Balg ist als Benutzerschnittstelle verwendbar, um einfache Eingaben zu ermöglichen. Alternativ oder zusätzlich kann die Anordnung aus Drucksensor, Fluidleitung und optional Balg eine Erfassung einer Kraft oder einer Änderung einer Kraft, beispielsweise einer Gewichtskraft ermöglichen. Alternativ oder zusätzlich kann mittels der Anordnung aus Drucksensor, Fluidleitung und Balg beispielsweise ein Blutdruck gemessen werden.

Bei einem tragbaren Funktionsmodul, wie es hier beschrieben ist, ist der erste Magnet insbesondere zwischen den elektrischen Kontakten der ersten Gruppe angeordnet.

Der erste Magnet ist insbesondere in einer geraden oder gekrümmten Reihe mit den elektrischen Kontakten der ersten Gruppe angeordnet.

Bei einem tragbaren Funktionsmodul, wie es hier beschrieben ist, ist der zweite Magnet insbesondere zwischen den elektrischen Kontakten der zweiten Gruppe angeordnet.

Der zweite Magnet ist insbesondere in einer geraden oder gekrümmten Reihe mit den elektrischen Kontakten der zweiten Gruppe angeordnet.

Bei einem tragbaren Funktionsmodul, wie es hier beschrieben ist, sind insbesondere die erste Gruppe elektrischer Kontakte zwischen dem ersten Magneten und einem dritten Magneten angeordnet und die zweite Gruppe elektrischer Kontakte zwischen dem zweiten Magneten und einem vierten Magneten angeordnet.

Alternativ ist lediglich ein Teil der elektrischen Kontakte der ersten Gruppe zwischen dem ersten Magneten und dem dritten Magneten angeordnet und/oder lediglich ein Teil der elektrischen Kontakte der zweiten Gruppe zwischen dem zweiten Magneten und dem vierten Magneten angeordnet.

Die beschriebenen Anordnungen der Magneten und der elektrischen Kontakte kann eine gleichmäßige und zuverlässige Kraftausübung der Magneten auf alle alle elektrischen Kontakte ermöglichen.

Bei einem tragbaren Funktionsmodul, wie es hier beschrieben ist, sind insbesondere der erste Magnet, die elektrischen Kontakte der ersten Gruppe und der dritte Magnet in einer Reihe angeordnet und der zweite Magnet, die elektrischen Kontakte der zweiten Gruppe und der vierte Magnet in einer Reihe angeordnet.

Objekte sind insbesondere dann in einer Reihe angeordnet, wenn sie auf einer geraden oder schwach gekrümmten Linie liegen. Der erste Magnet, die elektrischen Kontakte der ersten Gruppe und der dritte Magnet sind insbesondere in dieser Reihenfolge angeordnet. Der zweite Magnet, die elektrischen Kontakte der zweiten Gruppe und der vierte Magnet sind insbesondere in dieser Reihenfolge angeordnet.

Bei einem tragbaren Funktionsmodul, wie es hier beschrieben ist, ist die Reihe aus dem ersten Magneten, den elektrischen Kontakten der ersten Gruppe und gegebenenfalls dem dritten Magneten parallel zu der Reihe aus dem zweiten Magneten, den elektrischen Kontakten der zweiten Gruppe und gegebenenfalls dem vierten Magneten angeordnet.

Bei einem tragbaren Funktionsmodul, wie es hier beschrieben ist, sind die Magneten insbesondere so orientiert, dass nur bei einer einzigen vorbestimmten relativen Orientierung von Datenmodul und Funktionsmodul alle Paare je eines Magneten des Datenmoduls und eines korrespondierenden Magneten des Funktionsmoduls einander anziehen können.

Eine tragbare Vorrichtung umfasst ein tragbares Datenmodul, wie es hier beschrieben ist, und ein tragbares Funktionsmodul, wie es hier beschrieben ist.

Bei einem Verfahren zum Betreiben eines Datenmoduls oder einer tragbaren Vorrichtung mit einem Datenmodul und einem Funktionsmodul werden ein Messwert erfasst, geprüft, ob der Messwert eine vorbestimmte Bedingung erfüllt, wenn der Messwert die vorbestimmte Bedingung nicht erfüllt ein Parameter, der die Erfassung des Messwerts beeinflusst, geändert und die Erfassung des Messwerts wiederholt.

Bei einem Verfahren zum Betreiben eines Datenmoduls oder einer tragbaren Vorrichtung mit einem Datenmodul und einem Funktionsmodul werden ein Messwert erfasst, der Messwert ausgewertet, geprüft, ob das Ergebnis der Auswertung eine vorbestimmte Bedingung erfüllt, wenn das Ergebnis der Auswertung die vorbestimmte Bedingung nicht erfüllt ein Parameter, der zumindest entweder die Erfassung des Messwerts oder die Auswertung beeinflusst, geändert und zumindest entweder die Erfassung des Messwerts oder die Auswertung wiederholt.

Nachfolgend werden Ausführungsbeispiele anhand der beigefügten Figuren näher erläutert. Es zeigen:
- Figur 1: eine schematische perspektivische Darstellung einer tragbaren Vorrichtung mit einem Datenmodul und einem Funktionsmodul;
- Figur 2: eine schematische perspektivische Darstellung einer Variante der tragbaren Vorrichtung aus Figur 1;
- Figur 3: eine weitere schematische Darstellung der tragbaren Vorrichtung aus Figur 1;
- Figur 4: eine weitere schematische Darstellung der tragbaren Vorrichtung aus Figur 1;
- Figur 5: eine schematische perspektivische Darstellung des Datenmoduls aus den Figuren 1, 3, 4;
- Figur 6: eine weitere schematische perspektivische Darstellung des Datenmoduls aus den Figuren 1, 3, 4, 5;
- Figur 7: eine schematische perspektivische Darstellung des Funktionsmoduls aus den Figuren 1, 3, 4;
- Figur 8: eine weitere schematische perspektivische Darstellung des Funktionsmoduls aus den Figuren 1, 3, 4, 7;
- Figur 9: eine schematische perspektivische Darstellung eines weiteren Funktionsmoduls;
- Figur 10: ein schematisches Flussdiagramm eines Teils eines Verfahrens zum Erfassen von Messwerten;
- Figur 11: ein schematisches Flussdiagramm eines weiteren Teils des Verfahrens aus Figur 10;
- Figur 12: ein schematisches Flussdiagramm eines weiteren Teils des Verfahrens aus den Figuren 10 und 11;
- Figur 13: ein schematisches Flussdiagramm eines weiteren Teils des Verfahrens aus den Figuren 10 bis 12.

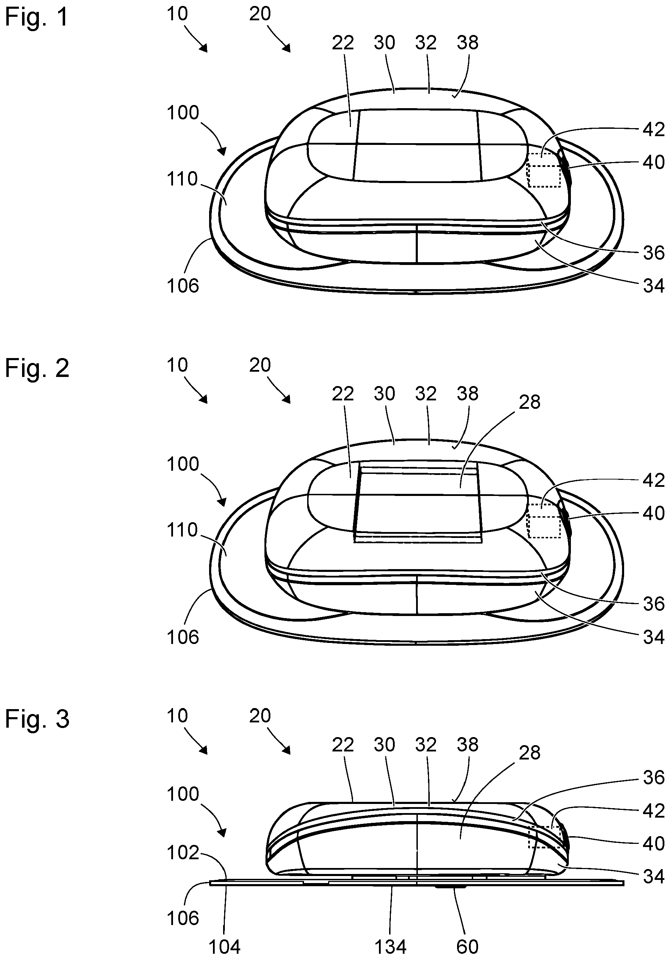

**Figur** 1 zeigt eine schematische perspektivische Darstellung einer tragbaren Vorrichtung 10 mit einem Datenmodul 20 und einem Funktionsmodul 100. Bei der in Figur 1 dargestellten Konfiguration sind das Datenmodul 20 und das Funktionsmodul 100 miteinander mechanisch und elektrisch verbunden, um zusammen die tragbare Vorrichtung 10 zu bilden.

Das **Datenmodul 20** weist eine näherungsweise flach quaderförmige, jedoch allseitig stark abgerundete Gestalt mit einer in Figur 1 sichtbaren Oberseite 22 und einer nicht sichtbaren, dem Funktionsmodul 100 zugewandten Unterseite.

Das Datenmodul 20 weist ein **Gehäuse 30** mit einem ersten Gehäuseteil 32 und einem zweiten Gehäuseteil 34 und einer Dichteinrichtung 36 zwischen dem ersten Gehäuseteil 32 und dem zweiten Gehäuseteil 34 auf. Das erste Gehäuseteil 32 bildet die Oberseite 22 des Datenmoduls 20. Das zweite Gehäuseteil 34 bildet die in Figur 1 nicht sichtbare, dem Funktionsmodul 100 zugewandte Unterseite des Datenmoduls 20. Die Gehäuseteile 32, 34 bilden zusammen mit der Dichteinrichtung 36 die äußere Oberfläche 38 des Gehäuses 30. Das Gehäuse 30 umschließt weitere Bestandteile des Datenmoduls 20 insbesondere fluiddicht oder hermetisch dicht.

Sowohl das erste Gehäuseteil 32 als auch das zweite Gehäuseteil 34 können jeweils weitgehend oder vollständig aus einem Kunststoff, Metall, Keramik oder einem anderen **Material** gebildet sein.

Das erste Gehäuseteil 32 und/oder das zweite Gehäuseteil 34 sind insbesondere aus einem für sichtbares Licht und optional auch für infrarotes und/oder für ultraviolettes Licht teilweise oder weitgehend oder im Wesentlichen vollständig **transparenten** Material, insbesondere Kunststoff gebildet.

Die zumindest teilweise transparente Ausgestaltung des Gehäuses 30 ermöglichte eine **Übertragung von Information** durch das Gehäuse 30 hindurch. Beispielsweise kann eine Statusanzeige mit einer oder mehreren Lichtquellen oder eine alphanumerische Anzeige oder eine graphische Anzeige innerhalb des Gehäuses Information darstellen, die von außen visuell sichtbar ist. Ferner kann ein Sender innerhalb des Gehäuses 30 mittels Licht Information digital oder analog an einen Empfänger außerhalb des Gehäuses 30 übertragen. Ferner kann ein Empfänger innerhalb des Gehäuses 30 Licht von einem Sender außerhalb des Gehäuses 30 empfangen, in dem Information analog oder digital codiert ist.

Die **Dichteinrichtung** 36 zwischen dem ersten Gehäuseteil 32 und dem zweiten Gehäuseteil 34 weist eine ringförmige Topologie auf. Die Dichteinrichtung 36 ist insbesondere aus Silikon, einem Elastomer oder einem anderen elastischen Kunststoff gebildet. Die Dichteinrichtung 36 liegt einerseits an einem Rand des ersten Gehäuseteils 32 und andererseits an einem Rand des zweiten Gehäuseteils 34 an. Wenn die Gehäuseteile 32, 34 durch eine oder mehrere Schraubverbindungen oder Rastverbindungen miteinander verbunden sind, wird die Dichteinrichtung 36 insbesondere zwischen gegenüberliegenden Rändern der Gehäuseteile 32, 34 gepresst oder gequetscht, so dass sie an den Rändern der Gehäuseteile 32, 34 vollständig anliegt. Alternativ oder zusätzlich kann die Dichteinrichtung 36 mit einem oder beiden Gehäuseteilen 32, 34 stoffschlüssig verbunden sein, beispielsweise durch Mehrkomponenten-Spritzguß oder durch Kleben.

Das Gehäuse weise einen **elastischen äußeren Oberflächenbereich** 40 auf, der einen Bereich 40 der äußeren Oberfläche 38 des Gehäuses 30 bildet. Bei dem dargestellten Beispiel ist der elastische äußere Oberflächenbereich 40 durch einen breiteren Bereich der Dichteinrichtung 36 gebildet. Der elastische äußere Oberflächenbereich 40 ist manuell elastisch verformbar, insbesondere indem er mittels eines darauf gelegten Fingers um eine kleine Strecke in Richtung des Inneren des Gehäuses 40 gedrückt wird.

Innerhalb des Gehäuses 30 ist nahe dem elastischen äußeren Oberflächenbereich 40 ein **Betätigungssensor** 42 vorgesehen. Da der Betätigungssensor 42 innerhalb des Gehäuses 30 angeordnet und deshalb abhängig vom Material des Gehäuses 30 nicht sichtbar ist, sind die Konturen des Betätigungssensors 42 in Figur 1 durch gestrichelte Linien angedeutet. Mittels des Betätigungssensors 42 kann eine elastische Verformung des elastischen äußeren Oberflächenbereichs 40 erfasst werden. Der Betätigungssensor 42 erzeugt ein analoges oder digitales und insbesondere elektrisches Signal, das den Betätigungszustand der elastischen äußeren Oberfläche 40 repräsentiert.

Der **Betätigungssensor** 42 umfasst beispielsweise einen elektrischen Taster oder Schalter, einen kapazitiven oder induktiven Sensor oder einen Magnetfeldsensor, wobei beispielsweise nahe dem elastischen äußeren Oberflächenbereich 40 ein Magnet in die Dichteinrichtung 36 eingebettet oder an dieser befestigt ist.

Das **Funktionsmodul** 100 ist an der in Figur 1 nicht sichtbaren Unterseite des Datenmoduls 20 angeordnet und mit dieser lösbar mechanisch verbunden.

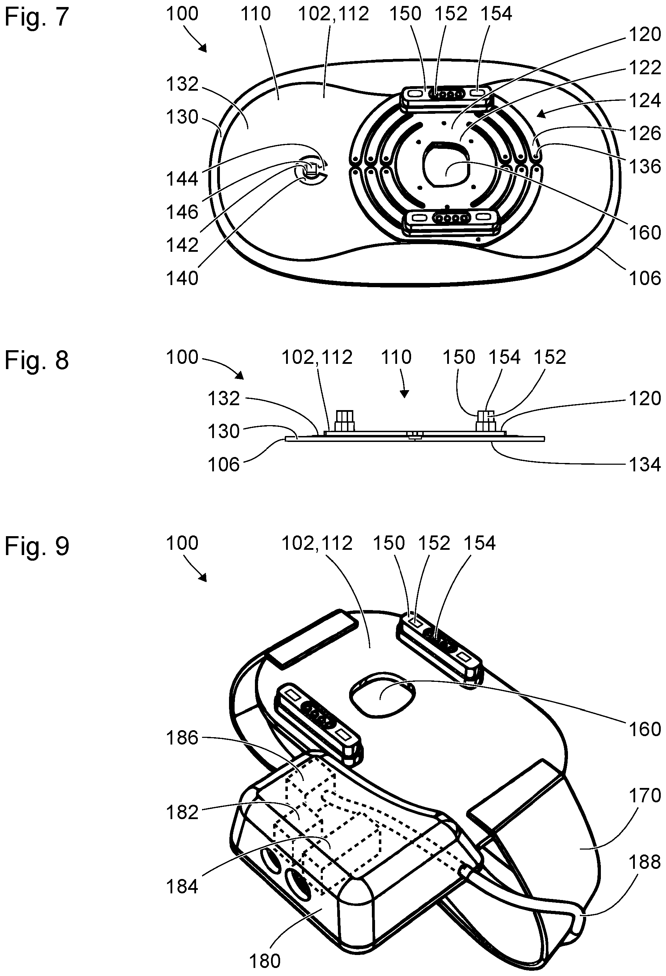

Das in Figur 1 dargestellte **Funktionsmodul** 100 weist eine flächige Gestalt mit einer in Figur 1 sichtbaren Oberseite 102 und einer in Figur 1 nicht sichtbaren Unterseite auf. Die Dicke des Funktionsmoduls 100 ist wesentlich geringer als seine Ausdehnung in zwei dazu senkrechten Richtungen. Der Rand 106 des Funktionsmoduls weist eine im Weiteren Sinne ovale Gestalt auf, beispielsweise die Gestalt einer Ellipse oder einer Superellipse oder eines Rechtecks mit abgerundeten Ecken. Das Funktionsmodul 100 ist anhand der Figuren 7 und 8 näher beschrieben.

**Figur 2** zeigt eine schematische perspektivische Darstellung einer Variante der anhand der Figur 1 dargestellten tragbaren Vorrichtung 10. Die Art der Darstellung, insbesondere die Perspektive auf die tragbare Vorrichtung 10, entspricht derjenigen der Figur 1.

Die in Figur 2 gezeigte tragbare Vorrichtung 10 unterscheidet sich von der anhand der Figur 1 dargestellten tragbaren Vorrichtung insbesondere dadurch, dass ein **Display** 28 in dem Gehäuse 30 angeordnet ist. Das erste Gehäuseteil 32 oder zumindest dessen die Oberseite 22 des Datenmoduls 20 bildender Bereich ist aus einem optisch transparenten Material gebildet. Dies ermöglicht die Betrachtung des Displays 28 und das Ablesen von darauf dargestellter Information.

Das **Display** 28 stellt eine Option dar, die für einige Anwendungen des Datenmoduls 20 und der tragbaren Vorrichtung 10 vorteilhaft ist. Im Folgenden beschriebene Merkmale, Eigenschaften und Funktionen können sowohl an einem Datenmodul 20 mit Display 28 als auch an einem Datenmodul ohne Display vorgesehen sein. Deshalb wird nachfolgend zwischen beiden Varianten in der Regel nicht unterschieden.

**Figur 3** zeigt eine weitere schematische Darstellung der tragbaren Vorrichtung 10 aus Figur 1 oder 2. Die Zeichenebene der Figur 3 ist orthogonal zu der Ebene, in der sich das Funktionsmodul 100 im Wesentlichen erstreckt, und orthogonal zu der Oberseite 22 und der dem Funktionsmodul 100 zugewandten Unterseite 24 des Datenmoduls 20.

In Figur 3 ist die **abgerundete Gestalt** des Datenmoduls 20 erkennbar. Ferner ist in der Zusammenschau der Figuren 2 und 3 erkennbar, dass die Dichteinrichtung 36 zwischen dem ersten Gehäuseteil 32 und dem zweiten Gehäuseteil 34 nicht in einer Ebene liegt oder sich parallel zu einer Ebene erstreckt.

In Figur 3 ist ferner die im Wesentlichen **flächige, ebene Gestalt** des Funktionsmoduls 100 erkennbar. An der dem Datenmodul 20 zugewandten Oberseite 102 des Funktionsmoduls 100 sind Einrichtungen zur lösbaren mechanischen und elektrischen Verbindung des Funktionsmoduls 100 mit dem Datenmodul erkennbar, die anhand der Figuren 7, 8 beschrieben sind. An der Unterseite 104 des Funktionsmoduls 100 ist eine Klebeschicht 134 vorgesehen, die beispielsweise eine Klebeverbindung mit einer Hautoberfläche eines Patienten ermöglicht.

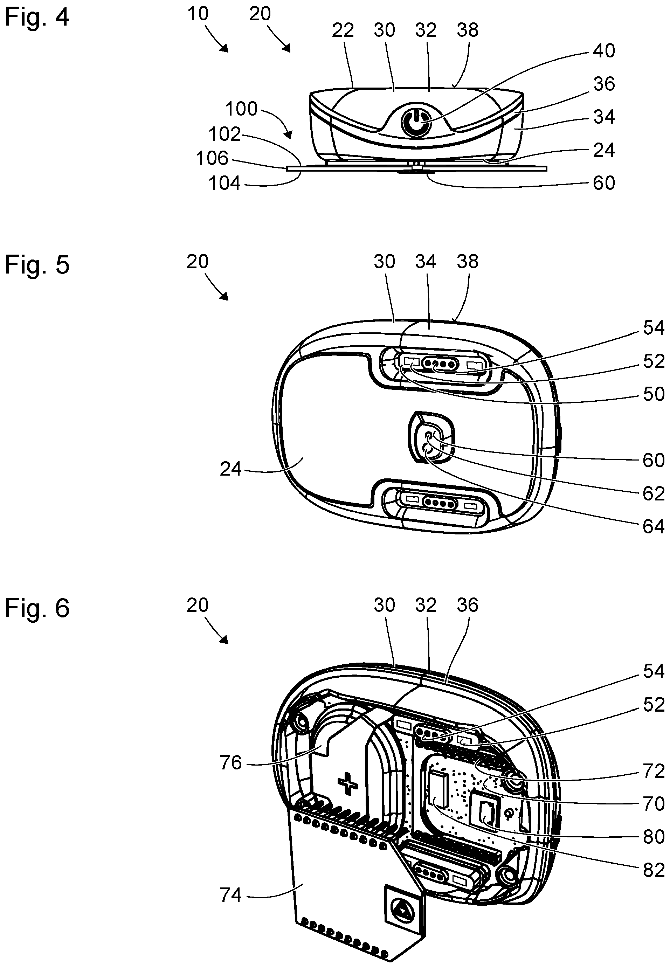

In Figur 3 ist ferner ein **konvexer äußerer Oberflächenbereich** 60 des Gehäuses 30 des Datenmoduls 20 erkennbar, der durch eine in Figur 3 an sich nicht sichtbare Öffnung des Funktionsmoduls 100 hindurch ragt und anhand der Figur 5 beschrieben ist.

**Figur 4** zeigt eine weitere schematische Darstellung der tragbaren Vorrichtung 10 aus den Figur 1 bis 3. Die Zeichenebene Figur 3 ist orthogonal zu der Zeichenebene der Figur 3, orthogonal zu der Ebene, in der sich das Funktionsmodul 100 im Wesentlichen erstreckt, orthogonal zu der Oberseite 22 und zu der Unterseite 24 des Datenmoduls 20.

In Figur 4 ist der **elastische äußere Oberflächenbereich** 40 des Datenmoduls 20 erkennbar, der durch einen deutlich verbreiterten Bereich der Dichteinrichtung 36 zwischen den Gehäuseteilen 32, 34 gebildet ist. Bei dem dargestellten Beispiel ist der elastische äußere Oberflächenbereich 40 durch ein Symbol als Ein- und Ausschaltknopf gekennzeichnet. Das Symbol ist insbesondere farbig abgehoben und/oder als Relief dargestellt.

In Figur 4 ist ferner wie in Figur 3 der **konvexe äußere Oberflächenbereich** 60 des Datenmoduls 20 erkennbar, der durch eine in Figur 4 an sich nicht sichtbare Öffnung des Funktionsmoduls 100 hindurch ragt und anhand der Figur 5 beschrieben ist.

**Figur 5** zeigt eine schematische perspektivische Darstellung des Datenmoduls 20 der tragbaren Vorrichtung aus den Figuren 1 bis 4. In Figur 5 ist vor allem die in den Figuren 1 und 2 nicht sichtbare Unterseite 24 des Datenmoduls 20 erkennbar.

In der Zusammenschau der Figuren 1 bis 5 ist eine im Wesentlichen **spiegelsymmetrische** Gestalt der äußeren Oberfläche 38 des Gehäuses 30 erkennbar.

Die äußere Oberfläche 38 des Gehäuses 30 des Datenmoduls 20 weist an der Unterseite 24 des Datenmoduls zwei **konkave äußere Oberflächenbereiche** 50 auf, von denen nur einer mit Bezugszeichen versehen ist. Bei dem dargestellten Beispiel sind die konkaven äußeren Oberflächenbereiche 50 spiegelsymmetrisch und parallel zu einander in Randbereichen der Unterseite 24 des Datenmoduls 20 angeordnet. Jeder der konkaven äußeren Oberflächenbereiche 50 weist eine länglich rechteckige Gestalt mit böschungsartig geneigten Randbereichen auf.

An beiden Enden jedes konkaven äußeren Oberflächenbereichs 50 ist jeweils ein **Magnet** 52 vorgesehen. Die Magneten 52 können in die äußere Oberfläche 38 des Gehäuses 30 eingelassen sein, so dass Oberflächenbereiche der Magneten 52 Teile der konkaven äußeren Oberflächenbereiche 50 bilden. Alternativ sind die Magneten 52 unter den konkaven äußeren Oberflächenbereichen 50 angeordnet, also entweder in das Material des zweiten Gehäuseteils 34 eingebettet oder innerhalb des von dem Gehäuse 30 umschlossenen Raumbereichs angeordnet.

An jedem der beiden konkaven äußeren Oberflächenbereiche 50 sind zwischen den Magneten 52 jeweils mehrere **elektrische Kontakte** 54 angeordnet, die jeweils Teilbereiche der konkaven Oberflächenbereiche 50 bilden. Die elektrischen Kontakte 54 sind offen liegende Oberflächenbereiche metallischer oder anderer elektrisch leitfähiger Bauteile, die in das zweite Gehäuseteil 34 eingebettet sind. Bei dem dargestellten Beispiel sind die elektrischen Kontakte 54 jeweils in einer Reihe angeordnet.

In Figur 5 ist ferner der bereits in den Figuren 3 und 4 erkennbare **konvexe äußere Oberflächenbereich** 60 des Gehäuses 30 erkennbar. Der konvexe äußere Oberflächenbereich 60 ragt über die im Übrigen im Wesentlichen ebene Unterseite 24 des Datenmoduls vor. Der konvexe äußere Oberflächenbereich 60 weist eine nicht rotationssymmetrische Gestalt mit böschungsartig geneigten Flanken auf.

Der **konvexe äußere Oberflächenbereich** 60 ist dafür vorgesehen und ausgebildet, um durch eine Durchgangsöffnung in dem Funktionsmodul hindurch zu ragen und beispielsweise an der Hautoberfläche einer Patientin oder eines Patienten anzuliegen, um Messungen zu ermöglichen. Dazu weist der konvexe äußere Oberflächenbereich 60 einen oder mehrere Sensoren zum Erfassen der Körpertemperatur oder eines anderen Parameters, der die Atmung, die Herztätigkeit, die Tätigkeit des Gehirns, die Konzentration von Zucker im Blut oder eines anderen Stoffs in einer Körperflüssigkeit oder in Gewebe oder eine andere Eigenschaft oder Funktion des Körpers oder eines Organs charakterisiert, auf.

Bei dem dargestellten Beispiel umfasst der konvexe äußere Oberflächenbereich 60 eine **Lichtaustrittsfläche** 62, durch die Licht einer Lichtquelle des Datenmoduls 20 austreten kann, und eine **Lichteintrittsfläche** 64, durch die Licht in das Datenmodul 20 eintreten und auf einen Lichtsensor fallen kann. Dies kann beispielsweise eine optische Messung einer Sauerstoffsättigung oder eines Blutzuckerspiegels oder einer Konzentration eines anderen Stoffs in Blut oder Gewebe ermöglichen.

**Figur 6** zeigt eine weitere schematische perspektivische Darstellung des **Datenmoduls** 20 aus den Figuren 1 bis 5. Die Perspektive auf das Datenmodul 20 ähnelt derjenigen der Figur 5. In Unterschied zu der Darstellung in Figur 5 ist das Gehäuse 30 geöffnet dargestellt, nämlich das zweite Gehäuseteil 34 entfernt. Dies ermöglicht einen Blick auf die im Inneren des Gehäuses 30 angeordneten und nachfolgend beschriebenen Komponenten.

In dem Gehäuse 30 ist eine **Platine** 70 mit Leiterbahnen in einer oder mehreren Ebenen und elektronischen Bauteilen sowie einer durch **Steckkontakte** 72 gebildeten Schnittstelle vorgesehen. Bei dem dargestellten Beispiel sind die Steckkontakte 72 in zwei geraden Reihen angeordnet und entsprechen geometrisch, mechanisch und elektrisch einer Norm für elektronische Kommunikationsmodule.

In dem Gehäuse 30 kann ferner ein **Kommunikationsmodul** 74 angeordnet sein, das geometrisch, mechanisch und elektrisch zu der durch die Steckkontakte 72 gebildeten Schnittstelle kompatibel ist. Das Kommunikationsmodul 74 ist in Figur 6 in der für eine Verbindung mit den Steckkontakten 72 erforderlichen Orientierung, aber von den Steckkontakten 72 beabstandet dargestellt, um den Blick auf weitere Bauteile zu ermöglichen. Das Kommunikationsmodul ermöglicht beispielsweise eine Kommunikation per WLAN (insbesondere gemäß einem Standard der IEEE-802.11-Familie), Bluetooth (insbesondere gemäß dem Standard IEEE 802.15.1) und/oder ZigBee (insbesondere gemäß dem Standard IEEE 802.15.4).

In dem Gehäuse 30 ist ferner eine **Leistungsquelle** 76 angeordnet, beispielsweise ein Kondensator, eine nicht wiederaufladbare Primärbatterie, ein wiederaufladbarer Akkumulator oder eine Brennstoffzelle. Die Leistungsquelle 76 ist dafür vorgesehen und ausgebildet, elektrische Leistung für Bauteile innerhalb des Datenmoduls 20 einschließlich das Kommunikationsmoduls 74 und optional auch für ein mit dem Datenmodul 20 verbundenes Funktionsmodul bereitzustellen.

Das Datenmodul 20 umfasst ferner einen **Prozessor** 80 zur insbesondere digitalen elektronischen Verarbeitung von Daten. Der Prozessor kann wie in Figur 6 gezeigt unmittelbar mit der Platine 70 verbunden oder an einer separaten Platine angeordnet sein.

Das Datenmodul 20 umfasst ferner einen **Beschleunigungssensor** 82 zum Erfassen einer Beschleunigung und zum Erzeugen eines Signals, das die erfasste Beschleunigung repräsentiert. Der Beschleunigungssensor 82 kann wie in Figur 6 angedeutet als separates elektronisches Bauteil an der Platine 70 angeordnet sein. Alternativ und abweichend von der Darstellung in Figur 6 kann der Beschleunigungssensor 82 beispielsweise mit dem Prozessor 80 integriert sein.

Der **Beschleunigungssensor** 82 ist insbesondere vorgesehen und ausgebildet zum Erfassen einer linearen Beschleunigung in einer, zwei oder drei Richtungen. Alternativ oder zusätzlich kann der Beschleunigungssensor 82 vorgesehen und ausgebildet sein zum Erfassen einer Drehrate um eine, zwei oder drei Achsen.

Wenn der Beschleunigungssensor am Brustkorb einer Person befestigt ist, kann der Beschleunigungssensor beispielsweise das Erfassen von Bewegungen des Brustkorbs aufgrund von **Atmung** und/oder **Herzschlag** ermöglichen. Ferner kann der Beschleunigungssensor das Erfassen der Orientierung des Datenmoduls und damit auch einer Person, an der das Datenmodul befestigt ist, im Raum ermöglichen. Ferner kann der Beschleunigungssensor das Erfassen eines Sturzes oder eines anderen Unfalls einer Person, an der das Datenmodul befestigt ist, ermöglichen.

**Figur 7** zeigt eine weitere schematische perspektivische Darstellung des **Funktionsmoduls** 100 der anhand der Figuren 1 bis 4 dargestellten tragbaren Vorrichtung 10. Die Art der Darstellung, insbesondere die Perspektive auf das Funktionsmodul 100, entspricht derjenigen der Figur 1. In Figur 7 ist die gesamte bei der vorgesehenen Verwendung dem Datenmodul zugewandte Oberseite 102 des Funktionsmoduls 100 sichtbar.

Das **Funktionsmodul** 100 weist wie erwähnt eine flächige, im Wesentlichen ebene Gestalt mit der in Figur 7 sichtbaren Oberseite 102, der in den Figuren 3, 4 bezeichneten Unterseite 104 und einem Rand 106 auf. Der Rand 106 des Funktionsmoduls 100 ist bei dem dargestellten Beispiel vollständig konvex, nämlich im weiteren Sinne oval, und symmetrisch zu mindestens einer Spiegelebene. In der Ebene weist das Funktionsmodul in einer ersten Richtung, die nachfolgend als Längsrichtung bezeichnet ist, eine maximale Erstreckung und in einer zweiten Richtung, die orthogonal zu der Längsrichtung und nachfolgend als Querrichtung bezeichnet ist, eine minimale Erstreckung auf.

Das Funktionsmodul 100 weist eine Tragstruktur 110 mit einer **strukturieren Schicht** 120 und einer **unstrukturierten Schicht** 130 auf. Die strukturierte Schicht 120 weist einen Zentralbereich 122 auf, der sich im Wesentlichen in der Querrichtung des Funktionsmoduls erstreckt. An den Zentralbereich 122 anschließend weist die strukturierte Schicht 120 mehrere kammartige Strukturen 124 auf. Bei dem dargestellten Beispiel sind vier kammartige Strukturen 124 spiegelsymmetrisch zu einer Spiegelebene in Längsrichtung des Funktionsmoduls 100 und spiegelsymmetrisch zu einer Spiegelebene in Querrichtung des Funktionsmoduls 100.

Jede **kammartige Struktur** 124 umfasst mehrere zinken- oder fingerförmige Bereiche, hier Finger 126 genannt. Bei dem dargestellten Beispiel sind alle Finger 126 kreisbogenförmig und innerhalb einer kammartigen Struktur untereinander parallel.

Der Rand der **unstrukturierten Schicht** 130 bildet den Rand des Funktionsmoduls. Die unstrukturierte Schicht 130 ist insbesondere aus einem elastischeren Material gebildet als die strukturierte Schicht 120. Beispielsweise ist die unstrukturierte Schicht 130 aus einem Schaumstoff gebildet. An der in Figur 7 abgewandten Unterseite der unstrukturierten Schicht 130 ist die erwähnte Klebeschicht 134 (vgl. Figuren 3, 4) vorgesehen, die die Unterseite des Funktionsmoduls bildet.

Zwischen der strukturierten Schicht 120 und der unstrukturierten Schicht 130 ist eine **Zwischenschicht** 132 vorgesehen. Die Dicke der Zwischenschicht 132 ist deutlich geringer als die Dicke der strukturierten Schicht 120 und die Dicke der unstrukturierten Schicht 130. Die geringe Dicke der Zwischenschicht 132 schafft eine große Biegeelastizität der Zwischenschicht 132.

Die Zwischenschicht 132 weist eine längliche, abgerundete und taillierte Gestalt auf. Die strukturierte Schicht 120 ist an der schmalsten Stelle der Zwischenschicht 132 angeordnet und weist dort mit dieser bündige Randabschnitte auf.

Die **strukturierte Schicht** 120 liegt vollflächig an der **Zwischenschicht** 132 an, das heißt alle Oberflächenbereiche der strukturierten Schicht 120, die zu der Zwischenschicht 132 parallel und dieser zugewandt sind, liegen an der Zwischenschicht 132 an oder haben allenfalls einen geringen Abstand von dieser.

Bei dem dargestellten Beispiel sind die strukturierte Schicht 120 und die Zwischenschicht 132 durch **Befestigungspunkte** 136 mit einander mechanisch verbunden. An dem freien Ende jedes Fingers 126 der strukturierten Schicht 120 ist ein Befestigungspunkt 136 angeordnet. Jeder Befestigungspunkt wird beispielsweise durch eine punktförmige Schweiß- oder Klebeverbindung gebildet.

Alternativ oder zusätzlich kann die strukturierte Schicht 120 vollflächig stoffschlüssig mit der Zwischenschicht 132 verbunden sein.

Die **unstrukturierte Schicht** 130 und die **Zwischenschicht** 132 sind insbesondere vollflächig mit einander stoffschlüssig verbunden, beispielsweise durch eine Klebung oder eine Schweißverbindung. Das heißt, der gesamte Oberflächenbereich der Zwischenschicht, der zu der unstrukturierten Schicht 130 parallel und ihr zugewandt ist, ist mit der unstrukturierten Schicht 130 schoffschlüssig verbunden.

Wenn eine Person, die die tragbare Vorrichtung 10 (vgl. Figuren 1 bis 4) an der Hautoberfläche trägt, sich bewegt, können mechanische **Schwingungen** entstehen oder aufgebaut werden, die die Klebeverbindung zwischen dem Funktionsmodul und der Haut lösen und vor allem die Gültigkeit erfasster Messwerte beeinträchtigen können.

Die innere Struktur der Tragstruktur 110 kann solche **Schwingungen dämpfen** oder ihren Aufbau unterdrücken. Dazu trägt insbesondere der mehrschichtige Aufbau mit Schicht aus unterschiedlichen Materialien mit unterschiedlichen mechanischen Eigenschaften bei. Ferner können zur Dämpfung oder Unterdrückung von Schwingungen die mechanischen Eigenschaften der beteiligten Materialien beitragen. Ferner kann Reibung zwischen nicht vollflächig mechanisch verbundenen Schichten zur Dämpfung oder Unterdrückung von Schwingungen beitragen. Dies gilt bei dem dargestellten Beispiel insbesondere für die strukturierte Schicht 120 und die Zwischenschicht 132.

Optional und abweichend von der Darstellung anhand der Figur 7 kann die strukturierte Schicht 120 vollflächig stoffschlüssig mit der Zwischenschicht 132 verbunden sein.

Bei dem dargestellten Beispiel weist die Zwischenschicht 132 einen C-förmigen **Schlitz** 140 auf, also eine C-förmige Ausnehmung, die die Zwischenschicht 132 vollständig durchdringt. Dieser C-förmige Schlitz 140 kann durch Stanzen, Laserschneiden, Wasserstrahlschneiden, Ätzen oder auf andere Weise erzeugt sein. Bei dem dargestellten Beispiel weist der C-förmige Schlitz 140 die Gestalt eines fast vollständigen Kreisbogens auf. Die Breite des Schlitzes 140 beträgt beispielsweise ca. 2 mm bis 3 mm,

Der C-förmige Schlitz 140 umschließt eine halbinselartige Struktur 142 der Zwischenschicht 132, im Folgenden als **Halbinsel** 142 bezeichnet. Die Halbinsel 142 ist durch einen schmalen **Steg** 144 zwischen den einander zugewandten Enden des C-förmigen Schlitzes 140 mit der übrigen Zwischenschicht 132 verbunden. Die Halbinsel 142 weist beispielsweise einen Durchmesser von ca. 3 mm auf. Der Steg 144 weist beispielsweise eine Breite von ca. 1 mm bis 2 mm auf.

An der bei der Darstellung in Figur 7 der betrachtenden Person zugewandten Oberfläche der Halbinsel 142 - bei dem dargestellten Beispiel ein Teil der Oberseite 102 des Funktionsmoduls 100 - ist ein **Temperatursensor** 146 angeordnet. Der Temperatursensor 146 erfasst die Temperatur und erzeugt ein Sensorsignal, das die erfasste Temperatur repräsentiert.

Der Temperatursensor 146 ist insbesondere klein und weist eine geringe Masse auf. Der Temperatursensor 146 ist beispielsweise quaderförmig mit Kantenlängen von 1 mm bis 2 mm. Der Temperatursensor 146 kann auf die Oberfläche der Halbinsel 142 geklebt oder geschweißt oder auf andere Weise mit dieser gefügt sein.

An der bei der Darstellung in Figur 7 von der betrachtenden Person abgewandten Oberfläche der Halbinsel 142 - und deshalb in Figur 7 nicht sichtbar - ist ein **weiterer Temperatursensor** angeordnet. Dieser weitere Temperatursensor und der Temperatursensor 146 sind insbesondere baugleich. Beide Temperatursensoren sind insbesondere entlang einer Achse parallel zu einer Flächennormalen der Zwischenschicht 132 oder ihrer großen ebenen Oberflächenbereiche angeordnet. Beide Temperatursensoren sind insbesondere spiegelsymmetrisch zu einer Spiegelebene angeordnet, wobei die Spiegelebene in der Zwischenschicht 132 angeordnet und parallel zu deren ebenen oder im Wesentlichen ebenen Oberflächenbereichen ist.

Die Zwischenschicht 132 enthält insbesondere **elektrische Leitungen** zur Übertragung der von den Temperatursensoren 146 erzeugten Sensorsignale und optional auch zur Bereitstellung elektrischer Leistung für die Temperatursensoren 146. Dazu ist die Zwischenschicht 132 insbesondere als flexible Leiterplatte ausgebildet.

Der C-förmige Schlitz 140 reduziert die Übertragung von Wärme innerhalb der Zwischenschicht 132 und kann dadurch eine präzisere und weniger träge Temperaturmessung ermöglichen. Die Anordnung zweier Temperatursensoren 146 kann die Erfassung von Temperaturdifferenzen zwischen beiden Seiten der Zwischenschicht 132 und damit eine Korrektur eines systematischen Messfehlers ermöglichen.

Alternativ und abweichend von der obigen Darstellung kann nur ein einziger Temperatursensor vorgesehen sein, der insbesondere an der in der Darstellung der Figur 7 von der betrachtenden Person abgewandten Seite der Zwischenschicht angeordnet ist.

Die unstrukturierte Schicht 130 ist insbesondere auch im Bereich des C-förmigen Schlitzes 140 unstrukturiert, um eine unbeabsichtigte Beschädigung der Halbinsel 142 und der Temperatursensoren 146 und einen unangenehme Haptik zu vermeiden.

Das Funktionsmodul 100 weist ferner zwei **konvexe äußere Oberflächenbereiche** 150 auf. Diese sind bei dem dargestellten Beispiel jeweils im wesentlichen länglich rechteckig, parallel zu einander und spiegelsymmetrisch zu einer Symmetrieebene, die die Längsrichtung des Funktionsmoduls 100 enthält, an den Enden des Zentralbereichs 122 der strukturierten Schicht 120 angeordnet. Die konvexen äußeren Oberflächenbereiche 150 des Funktionsmoduls korrespondieren hinsichtlich Anordnung und Gestalt zu den konkaven äußeren Oberflächenbereichen 50 des Datenmoduls 20 (vgl. Figur 5).

Jeder konvexe äußere Oberflächenbereich 150 des Funktionsmoduls 100 weist an jedem Ende einen **Magneten** 152 und dazwischen eine gerade Reihe von elektrischen Kontakten 154 auf. Die Magneten 152 sind jeweils unter der konvexen äußeren Oberfläche 150 angeordnet oder in die Oberfläche eingelassen, bilden also Teile des konvexen äußeren Oberflächenbereichs 150.

Jeder konvexe äußere Oberflächenbereich 150 des Funktionsmoduls 100 weist zwischen den Magneten 152 jeweils eine gerade Reihe von **elektrischen Kontakten** 154 auf. Die elektrischen Kontakte 154 sind Oberflächenbereiche elektrisch leitfähiger Bauteile oder Bereiche des Funktionsmoduls 100, die Teile des konvexen äußeren Oberflächenbereichs 150 bilden. Die elektrischen Kontakte 154 sind insbesondere durch die Enden federbelasteter Kontaktstifte gebildet.

Das Funktionsmodul 100 weist eine **Öffnung** 160 auf, die die strukturierte Schicht 120, die Zwischenschicht 132 und die unstrukturierte Schicht 130 vollständig durchdringt. Die Öffnung 160 ist zwischen den konvexen äußeren Oberflächenbereichen 150 im Zentralbereich 122 der strukturierten Schicht 120 angeordnet. Die Kontur der Öffnung 160 weist keine Rotationssymmetrie auf und entspricht dem Querschnitt des konvexen äußeren Oberflächenbereichs 60 des Gehäuses 30 des Datenmoduls 20 (vgl. Figur 5). In genau einer relativen Orientierung des Datenmoduls 20 relativ zu dem Funktionsmodul 100 kann der konvexe äußere Oberflächenbereich 60 so weit in die Öffnung 160 eingreifen, dass er durch die Öffnung 160 hindurch ragt, wie dies in den Figuren 3, 4 angedeutet ist. In dieser relativen Orientierung können die konvexen äußeren Oberflächenbereiche 150 des Funktionsmoduls 100 in die konkaven äußeren Oberflächenbereiche 50 des Datenmoduls 20 eingreifen.

Bei der **vorgesehenen Verwendung** des Funktionsmoduls 100 zusammen mit einem Datenmodul 20 greifen die konvexen Oberflächenbereiche 150 in die korrespondierenden konkaven Oberflächenbereiche 50 des Gehäuses 30 des Datenmoduls 20 ein (vgl. Figur 5). Dabei ziehen die einander gegenüberliegenden Magneten 52, 152 einander an und halten das Datenmodul 20 an dem Funktionsmodul 100. Dadurch liegen die elektrischen Kontakte 54 des Datenmoduls 20 an den elektrischen Kontakten 154 des Funktionsmoduls an. Dies ermöglicht eine Übertragung elektrischer Leistung und elektrischer Signale zwischen dem Datenmodul und dem Funktionsmodul 100.

Gleichzeitig greift der konvexe äußere Oberflächenbereich 60 des Gehäuses 30 des Datenmoduls 20 durch die Öffnung 160 in dem Funktionsmodul 100 hindurch. Dadurch können die Lichtaustrittsfläche 62 und die Lichteintrittsfläche 64 an der Hautoberfläche einer Person, an der das Funktionsmodul festgeklebt ist, anliegen. Dies kann optische Messungen ermöglichen.

Die Anordnung der Magneten in zwei Gruppen, insbesondere an den Ecken eines ausgedehnten Rechtecks ermöglicht eine auch bei stärkerer dynamischer Belastung zuverlässige mechanische Verbindung. Die Anordnung jeweils einer Gruppe elektrischer Kontakte zwischen zwei Magneten ermöglicht eine zuverlässige Übertragung der magnetischen Anziehungskräfte auf die an einander anliegenden elektrischen Kontakte und damit eine zuverlässige Kontaktierung.

An der in Figur 7 nicht sichtbaren Unterseite 114 der Tragstruktur 110 können eine oder mehrere **Elektroden** vorgesehen sein, die kapazitive oder galvanische Messungen ermöglichen können.

**Figur 8** zeigt eine weitere schematische Darstellung des Funktionsmoduls aus den Figuren 1 bis 4 und 7. Die Zeichenebene der Figur 4 entspricht der Zeichenebene der Figur 3, ist also orthogonal zu der Zeichenebene der Figur 3, orthogonal zu der Ebene, in der sich das Funktionsmodul 100 im Wesentlichen erstreckt, orthogonal zu der Oberseite 102 und zu der Unterseite 104 des Funktionsmoduls 100.

In Figur 8 ist die weitgehend flache, im Wesentlichen ebene Gestalt der **Tragstruktur** 110 des Funktionsmoduls 100 erkennbar. An der Oberseite 102 des Funktionsmoduls 100 stehen die konvexen äußeren Oberflächenbereiche 150 gegenüber der Oberseite 112 der Tragstruktur 110 über.

In Figur 8 ist ferner erkennbar, dass die **Zwischenschicht** 132 wesentlich dünner ist als die strukturierte Schicht 120 und die unstrukturierte Schicht 130.

**Figur 9** zeigt eine schematische perspektivische Darstellung eines weiteren Funktionsmoduls 100.

Das in Figur 9 gezeigte **Funktionsmodul** 100 weist wie das anhand der Figuren 1 bis 4, 7 und 8 dargestellte Funktionsmodul eine Schnittstelle zum mechanischen und elektrischen Verbinden des Funktionsmoduls 100 mit einem Datenmodul, wie es anhand der Figuren 1 bis 6 beschrieben ist, auf. Die Schnittstelle umfasst insbesondere Magneten 152 und elektrische Kontakte 154 an zwei jeweils rechteckigen, parallel und spiegelsymmetrisch angeordneten konvexen äußeren Oberflächenbereichen 150.

Anders als das anhand der Figuren 1 bis 4, 7 und 8 dargestellte Funktionsmodul weist das in Figur 9 gezeigte Funktionsmodul 100 ein **Band** 170 auf, mittels dessen das Funktionsmodul beispielsweise am Unterarm oder am Handgelenk einer Person befestigt werden kann.

Ferner weist das Funktionsmodul 100 ein **Gehäuse** 180 auf, in dem eine Lichtquelle 182, eine Kamera 184 und ein Drucksensor 186 angeordnet sind.

Die **Lichtquelle** 182 erzeugt Licht zum Beleuchten oder Markieren eines Objekts, das mittels der Kamera 184 erfasst werden kann. Das von der Lichtquelle 182 erzeugte Licht kann teilweise oder vollständig in dem für das gesunde menschliche Auge sichtbaren Wellenlängenbereich oder teilweise oder vollständig im infraroten und/oder im ultravioletten Wellenlängenbereich liegen. Das von der Lichtquelle 182 erzeugte Licht kann die Erfassung eines Objekts mittels der Kamera 184 ermöglichen oder vereinfachen. Wenn das von der Lichtquelle 182 erzeugte Licht in dem für das gesunde menschliche Auge sichtbaren Spektralbereich liegt, kann es zusätzlich oder auch ausschließlich der Markierung des von der Kamera erfassten Bereichs dienen.

Die **Kamera** 184 ist insbesondere vorgesehen und ausgebildet zum Erfassen eines Codes, beispielsweise eines eindimensionalen Strichcodes ("Barcode"), eines zweidimensionalen Codes (beispielsweise QR-Code) und/oder von alphanumerischen Zeichen.

Der **Drucksensor** 186 ist mit einem Ende einer Fluidleitung 188 verbunden, um den Druck in der Fluidleitung 188 zu erfassen. Die Fluidleitung 188 kann den Drucksensor 186 mit einem Balg verbinden, der in den Querschnitt des Bands 170 eingebettet oder an der Innenseite des Bands 170 angeordnet ist. Alternativ kann die Fluidleitung 188 teilweise oder - abweichend von der Darstellung in Figur 9 - vollständig innerhalb des Querschnitts des Bands oder an der Innenseite des Bands 170, also innerhalb des von dem Band 170 umschlossenen Querschnitts angeordnet sein.

Durch Bewegungen des Handgelenks oder des Unterarms oder eines anderen Körperteils, um das das Band 170 des Funktionsmoduls 100 geschlungen ist, kann der Druck in der Fluidleitung 188 beeinflusst, insbesondere erhöht werden. Durch willkürliche Bewegungen erzeugte Druckänderungen in der Fluidleitung 188 können als Eingaben interpretiert werden. Auf diese Weise bilden der Drucksensor 186 und die Fluidleitung 188 eine Benutzerschnittstelle des Funktionsmoduls 100 und der tragbaren Vorrichtung 10.

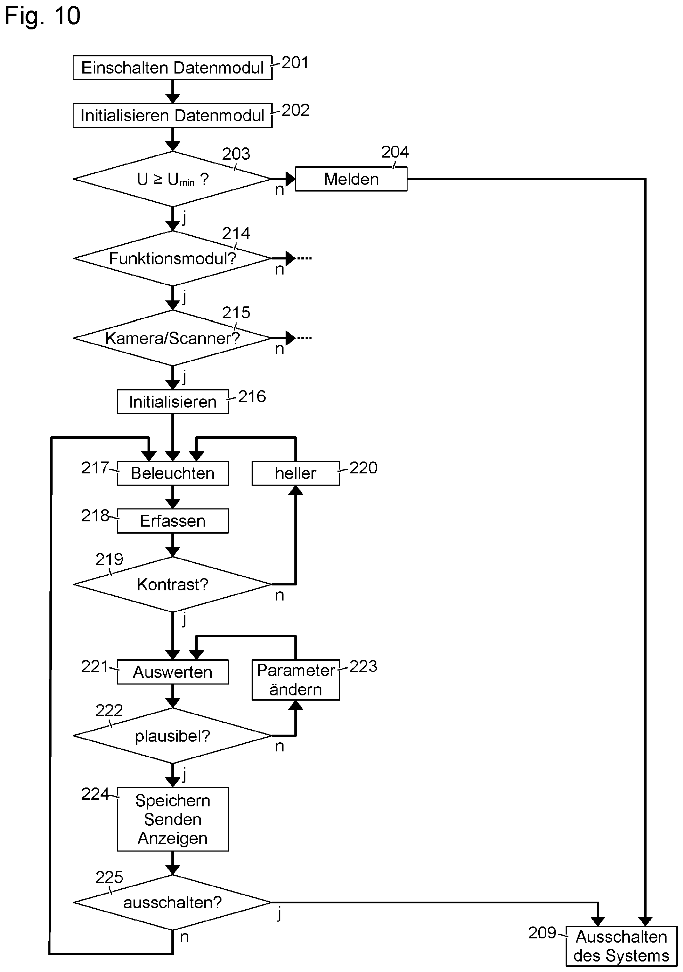

**Figur 10** zeigt ein schematisches Flussdiagramm eines Teils eines Verfahrens zum Betreiben eines Datenmoduls oder einer tragbaren Vorrichtung mit einem Datenmodul und einem Funktionsmodul. Das Verfahren ist auch von oder mit einem Datenmodul oder einer tragbaren Vorrichtung durchführbar, das bzw. die von dem anhand der Figuren 1 bis 9 Dargestellten abweichende Merkmale, Eigenschaften und Funktionen aufweist. Trotzdem sind nachfolgend zur Illustration Bezugszeichen aus den Figuren 1 bis 9 verwendet.

Bei einem ersten Schritt **201** wird das Datenmodul 20 eingeschaltet. Dies erfolgt beispielsweise durch einfaches oder mehrfaches manuelles Drücken eines elastischen Oberflächenbereichs 40 des Gehäuses 30 des Datenmoduls 20 während einer Zeitspanne vorbestimmter Mindestdauer. Alternativ wird das Datenmodul beispielsweise durch Schütteln oder Drehen oder mittels einer anderen Gesten eingeschaltet.

Bei einem folgenden Schritt **202** wird das Datenmodul initialisiert. Das Initialisieren umfasst beispielsweise das Starten eines Betriebssystems und eine Selbstdiagnose. Beim Initialisieren kann ferner geprüft werden, ob und welches Funktionsmodul 100 mit dem Datenmodul 20 verbunden ist.

Bei einem folgenden Schritt **203** wird geprüft, ob die - insbesondere von einem Akkumulator bereitgestellte - Versorgungsspannung U einen Mindestwert Uₘᵢₙ aufweist. Der dritte Schritt 203 kann abweichend von der Darstellung in Figur 10 vor dem Schritt 202 des Initialisierens oder gleichzeitig mit diesem oder während des Schritts 202 ausgeführt werden.

Wenn die Versorgungsspannung U nicht den Mindestwert Uₘᵢₙ aufweist, wird bei einem folgenden Schritt **204** an einer Benutzerschnittstelle 28 gemeldet, dass die Versorgungsspannung zu gering ist und angekündigt, dass das Datenmodul 20 ausgeschaltet werden wird. Die Meldung erfolgt insbesondere alphanumerisch, graphisch oder akustisch.

Nach einer vorbestimmten Zeitdauer wird bei einem folgenden Schritt **209** das Datenmodul 20 ausgeschaltet. Beim Ausschalten wird das Datenmodul insbesondere aus einem aktiven Modus in einen Energiesparmodus mit sehr geringer Leistungsaufnahme versetzt. Alternativ kann die Leistungsversorgung für das Datenmodul 20 ganz beendet werden. In jedem Fall wird insbesondere auch die Leistungsversorgung eines mit dem Datenmodul 20 verbundenen Funktionsmoduls 100 beendet oder reduziert. Wenn das Datenmodul 20 also Teil einer tragbaren Vorrichtung aus dem Datenmodul 20 und einem Funktionsmodul 100 ist, wird also insbesondere die gesamte tragbare Vorrichtung abgeschaltet.

Wenn die Versorgungsspannung U den Mindestwert Uₘᵢₙ aufweist oder überschreitet, wird bei einem folgenden Schritt **214** geprüft, ob ein Funktionsmodul 100 mit dem Datenmodul 20 verbunden ist. Dies erfolgt beispielsweise durch Abfragen einer Schnittstelle 54, mit der ein Funktionsmodul 100 gekoppelt sein kann. Alternativ erfolgt die Prüfung beispielsweise durch Auslesen eines Speichers oder Registers, in dem beispielsweise während des Initialisierens 202 des Datenmoduls 20 die Information abgelegt wurde, ob und welches Funktionsmodul 100 mit dem Datenmodul 20 verbunden ist.

Wenn ein Funktionsmodul 100 mit dem Datenmodul 20 verbunden ist, wird bei einem nächsten Schritt **215** geprüft, ob das Funktionsmodul 100 eine Kamera 184 oder einen Scanner zum Erfassen eines ein- oder mehrdimensionalen Codes, beispielsweise eines Strichcodes, eines QR-Codes oder alphanumerischer Zeichen umfasst.

Wenn das Funktionsmodul 100 eine Kamera 184 oder einen Scanner umfasst, wird bei einem folgenden Schritt **216** die Kamera oder der Scanner initialisiert. Dabei wird beispielsweise ein Betriebssystem der Kamera 184 oder des Scanners gestartet und eine Selbstdiagnose durchgeführt.

Bei einem folgenden Schritt **217** wird eine Lichtquelle zum Beleuchten eines mittels der Kamera 184 erfassten Objekts oder ein Laser oder eine andere Lichtquelle eines Scanners eingeschaltet, also mit Leistung versorgt, damit die Lichtquelle Licht erzeugt. Das Licht kann kontinuierlich oder gepulst erzeugt werden.

Bei einem folgenden Schritt **218** wird das beleuchtete Objekts mittels der Kamera erfasst oder von dem Objekt reflektiertes oder remittiertes Licht der Lichtquelle des Scanners mittels eines Lichtsensors erfasst.

Bei einem folgenden Schritt **219** wird der Kontrast in dem von der Kamera 184 erfassten Bild oder in dem von dem Scanner erfassten Signal geprüft. Wenn der Kontrast zu gering ist, wird bei einem folgenden Schritt **220** der Lichtquelle mehr Leistung zugeführt, um das Objekt heller zu beleuchten.

Die Schritte 217, 218, 219, 220 können einmal oder mehrfach wiederholt werden bis der Kontrast ausreichend ist oder eine maximale Helligkeit der Lichtquelle erreicht ist oder eine vorbestimmte Höchstzahl von Iterationen erreicht ist.

Abweichend von der Beschriftung in Figur 10 kann auch bei einem zu hohen Kontrast eine Maßnahme erfolgen, beispielsweise die der Lichtquelle zugeführte Leistung reduziert werden. Abweichend von der Darstellung in Figur 10 können ferner weitere Parameter des Beleuchtens und Erfassens geändert werden, beispielsweise die Wellenlänge, eine Zeitabhängigkeit des Beleuchtens, eine Brennweite der Kamera 184, eine Größe des gescannten Bereichs.

Bei einem folgenden Schritt **221** wird das von der Kamera 184 erfasste Bild oder das mittels des Scanners erfasste Muster ausgewertet. Dabei wird beispielsweise in einem eindimensionalen Strichcode oder in einem QR-Code oder einem anderen mehrdimensionalen Code codierte Information ermittelt oder mittels OCR in alphanumerischen Zeichen codierte Information ermittelt.

Bei einem folgenden Schritt **222** wird die Plausibilität des Ergebnisses der Decodierung geprüft. Dazu wird beispielsweise eine Prüfsumme gebildet oder die erhaltene Information mit einer Datenbank abgeglichen.

Wenn das Ergebnis nicht plausibel ist, beispielsweise die Prüfsumme nicht stimmt, wird bei einem folgenden Schritt **223** ein Parameter geändert, der auf die Auswertung im Schritt 221 Einfluss nimmt. Beispiele für einen solchen Parameter sind Filtereigenschaften oder Schwellenwerte.

Die Schritte 221, 222, 223 können einmal oder mehrfach wiederholt werden bis das Ergebnis plausibel oder eine vorbestimmte Höchstzahl von Iterationen erreicht ist.

Wenn das Ergebnis plausibel ist, wird es bei einem folgenden Schritt **224** in einem Speicher des Datenmoduls 20 gespeichert, von dem Datenmodul 20 an einen externen Empfänger gesendet und/oder beispielsweise an einer Benutzerschnittstelle 28 angezeigt.

Bei einem folgenden Schritt **225** wird geprüft, ob das Datenmodul ausgeschaltet werden soll. Dazu wird beispielsweise eine Benutzerschnittstelle 40 abgefragt oder geprüft, ob die Versorgungsspannung U noch einen Mindestwert Uₘᵢₙ aufweist oder überschreitet.

Wenn das Datenmodul ausgeschaltet werden soll, wird es bei dem bereits anhand der Figur 10 beschriebenen Schritt 209 ausgeschaltet.

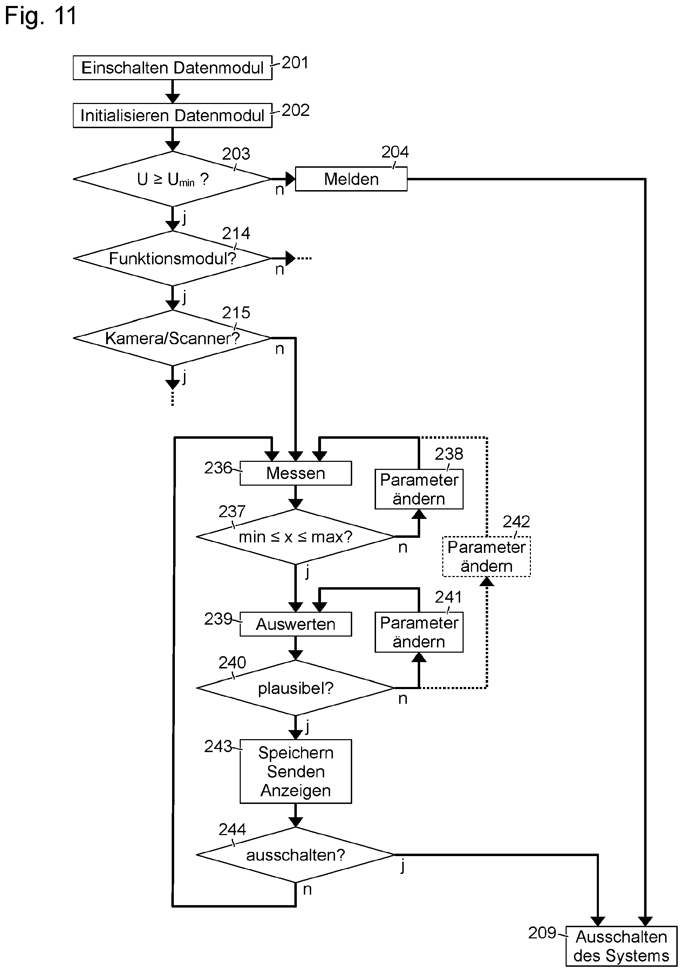

**Figur 11** zeigt ein schematisches Flussdiagramm eines weiteren Teils des bereits teilweise anhand des Flussdiagramms aus Figur 10 dargestellten Verfahrens.

Bei dem dargestellten Beispiel können genau zwei verschiedene Funktionsmodule 100 mit dem Datenmodul 20 verbunden sein. Das eine Funktionsmodul umfasst eine Kamera 184 oder einen Scanner. Das andere Funktionsmodul umfasst keine Kamera und keinen Scanner, stattdessen beispielsweise Elektroden, die dafür vorgesehen sind, an der Oberfläche der Haut einer Person anzuliegen, um kapazitive oder ohmsche Messungen elektrischer Spannungen oder anderer elektrischer Parameter zu ermöglich.

Wenn bei dem Schritt 215 festgestellt wurde, dass das Funktionsmodul 100 keine Kamera und keinen Scanner umfasst, erfolgt bei dem folgenden Schritt **236** eine Messung. Gemessen werden beispielsweise eine oder mehrere zeitabhängige elektrische Spannungen, um ein EKG (Elektrokardiogramm) oder ein EEG (Elektroenzephalogramm) zu erstellen.

Bei einem folgenden Schritt **237** wird geprüft, ob das Messergebnis vorbestimmte Bedingungen erfüllt, insbesondere in einem vorbestimmten Bereich liegt. In Figur 11 ist beispielhaft die Prüfung angedeutet, ob ein einzelner skalarer Parameter x in einem eindimensionalen Bereich mit einer Untergrenze min und einer Obergrenze max liegt. Ebenso ist eine komplexere Prüfung mit mehr und/oder komplexeren Bedingungen möglich.

Wenn das Messergebnis die vorbestimmten Bedingungen nicht erfüllt, werden bei einem folgenden Schritt **238** ein oder mehr Parameter geändert, die auf die Messung in Schritt 236 Einfluss nehmen. Beispiele für einen solchen Parameter sind Filtereigenschaften oder Schwellenwerte.

Die Schritte 236, 237, 238 können einmal oder mehrfach wiederholt werden bis das Messergebnis die vorbestimmten Bedingungen erfüllt oder eine vorbestimmte Höchstzahl von Iterationen erreicht ist.

Bei einem folgenden Schritt **239** wird das Messergebnis ausgewertet, um beispielsweise ein EKG oder ein EEG zu erstellen.

Bei einem folgenden Schritt **240** wird die Plausibilität des Ergebnisses der Auswertung geprüft. Beispielweise wird geprüft, ob ein aus dem EKG berechenbarer Puls in einem vorbestimmten Intervall liegt.

Wenn das Ergebnis der Auswertung nicht plausibel ist, wird bei einem folgenden Schritt **241** ein Parameter geändert, der die Auswertung beeinflusst, beispielsweise ein Schwellenwert oder ein Filterparameter.

Die Schritte 239, 240, 241 können einmal oder mehrfach wiederholt werden bis das Ergebnis der Auswertung plausibel ist oder eine vorbestimmte Höchstzahl von Iterationen erreicht ist.

Alternativ wird wie in Figur 11 in gestrichelten Linien angedeutet nach einem Schritt 242, in dem ein oder mehrere Parameter geändert werden, der Schritt 236 des Messens wiederholt.

In diesem Fall können die Schritte 236, 237, 239, 240, 241 einmal oder mehrfach wiederholt werden bis das Ergebnis der Auswertung plausibel ist oder eine vorbestimmte Höchstzahl von Iterationen erreicht ist. Auch eine Mischung oder Kombination der in durchgezogenen Linien angedeuteten Schleifen und der in gestrichelten Linien angedeuteten Schleife ist möglich. Dabei können weitere Bedingungen geprüft werden, von deren Erfüllung abhängig ist, welche Parameter geändert werden.

Wenn das Ergebnis plausibel ist, wird es bei einem folgenden Schritt **243** in einem Speicher des Datenmoduls 20 gespeichert, von dem Datenmodul 20 an einen externen Empfänger gesendet und/oder beispielsweise an einer Benutzerschnittstelle 28 angezeigt.

Bei einem folgenden Schritt **244** wird geprüft, ob das Datenmodul ausgeschaltet werden soll. Dazu wird beispielsweise eine Benutzerschnittstelle 40 abgefragt oder geprüft, ob die Versorgungsspannung U noch einen Mindestwert Uₘᵢₙ aufweist oder überschreitet.

Wenn das Datenmodul ausgeschaltet werden soll, wird es bei dem bereits anhand der Figur 10 beschriebenen Schritt 209 ausgeschaltet.

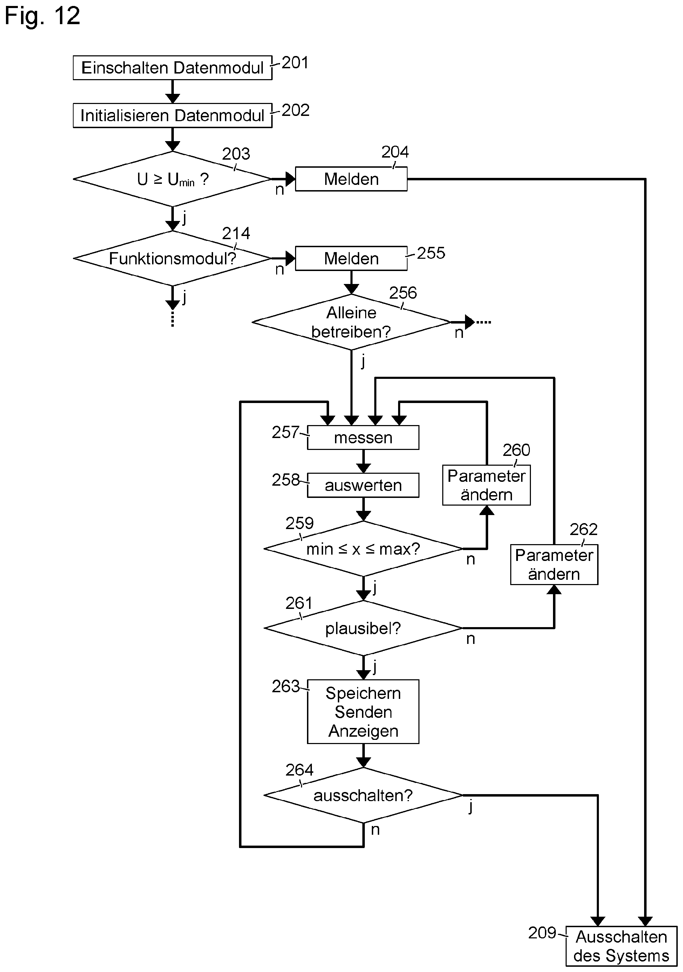

**Figur 12** zeigt ein schematisches Flussdiagramm eines weiteren Teils des bereits teilweise anhand der Flussdiagramme aus den Figuren 10 und 11 dargestellten Verfahrens.

Wenn im Schritt 214 festgestellt wurde, dass kein Funktionsmodul mit dem Datenmodul 20 verbunden ist, wird bei einem folgenden Schritt **255** an einer Benutzerschnittstelle 28 gemeldet, dass kein Funktionsmodul mit dem Datenmodul 20 verbunden ist. Die Meldung erfolgt insbesondere alphanumerisch, graphisch oder akustisch.

Bei einem folgenden Schritt **256** wird abgefragt, ob das Datenmodul 20 ohne Funktionsmodul betrieben werden soll.

Wenn das Datenmodul 20 ohne Funktionsmodul betrieben werden soll, erfolgt bei dem folgenden Schritt **257** eine Messung mittels eines Sensors des Datenmoduls 20, beispielsweise eine optische Messung der Sauerstoffsättigung oder eine Messung des Herzschlags oder der Atemtätigkeit mittels eines Beschleunigungssensors 82 des Datenmoduls 20.

Bei einem folgenden Schritt **258** wird das Messergebnis ausgewertet.

Bei einem folgenden Schritt **259** wird geprüft, ob das Ergebnis der Auswertung vorbestimmte Bedingungen erfüllt, insbesondere in einem vorbestimmten Bereich liegt. In Figur 12 ist beispielhaft die Prüfung angedeutet, ob ein einzelner skalarer Parameter x in einem eindimensionalen Bereich mit einer Untergrenze min und einer Obergrenze max liegt. Ebenso ist eine komplexere Prüfung mit mehr und/oder komplexeren Bedingungen möglich.

Wenn das Ergebnis der Auswertung die vorbestimmten Bedingungen nicht erfüllt, werden bei einem folgenden Schritt **260** ein oder mehr Parameter geändert, die auf die Messung in Schritt 257 oder auf die Auswertung in Schritt 258 Einfluss nehmen. Beispiele für solche Parameter sind die Helligkeit einer Lichtquelle, die Wellenlänge oder die Zeitabhängigkeit des von der Lichtquelle erzeugten Lichts, Filtereigenschaften oder Schwellenwerte.

Die Schritte 257, 258, 259, 260 können einmal oder mehrfach wiederholt werden bis das Ergebnis der Auswertung die vorbestimmten Bedingungen erfüllt oder eine vorbestimmte Höchstzahl von Iterationen erreicht ist.

Bei einem folgenden Schritt **261** wird die Plausibilität des Ergebnisses der Auswertung geprüft. Beispielweise wird geprüft, ob eine Sauerstoffsättigung in einem bei einer lebenden Person zu erwartenden vorbestimmten Intervall liegt.

Wenn das Ergebnis der Auswertung nicht plausibel ist, werden bei einem folgenden Schritt **262** ein oder mehr Parameter geändert, die auf die Messung in Schritt 257 oder auf die Auswertung in Schritt 258 Einfluss nehmen. Beispiele für solche Parameter sind die Helligkeit einer Lichtquelle, die Wellenlänge oder die Zeitabhängigkeit des von der Lichtquelle erzeugten Lichts, Filtereigenschaften oder Schwellenwerte.

Die Schritte 257, 258, 259, 260, 261, 262 können einmal oder mehrfach wiederholt werden bis das Ergebnis der Auswertung plausibel ist oder eine vorbestimmte Höchstzahl von Iterationen erreicht ist.

Wenn das Ergebnis plausibel ist, wird es bei einem folgenden Schritt **263** in einem Speicher des Datenmoduls 20 gespeichert, von dem Datenmodul 20 an einen externen Empfänger gesendet und/oder beispielsweise an einer Benutzerschnittstelle 28 angezeigt.

Bei einem folgenden Schritt **264** wird geprüft, ob das Datenmodul ausgeschaltet werden soll. Dazu wird beispielsweise eine Benutzerschnittstelle 40 abgefragt oder geprüft, ob die Versorgungsspannung U noch einen Mindestwert Uₘᵢₙ aufweist oder überschreitet.

Wenn das Datenmodul ausgeschaltet werden soll, wird es bei dem bereits anhand der Figur 10 beschriebenen Schritt 209 ausgeschaltet.

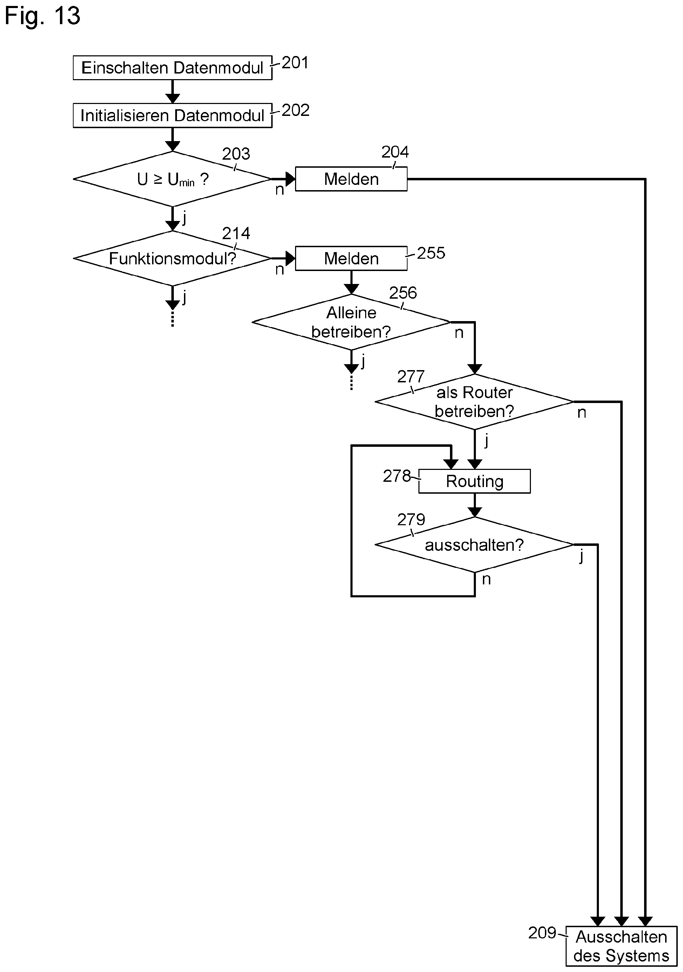

**Figur 13** zeigt ein schematisches Flussdiagramm eines weiteren Teils des bereits teilweise anhand der Flussdiagramme aus den Figuren 10 bis 12 dargestellten Verfahrens.

Wenn im Schritt 256 festgestellt wurde, dass das Datenmodul 20 nicht allein betrieben werden soll, wird bei einem folgenden Schritt **277** abgefragt, ob das Datenmodul 20 als Router verwendet werden soll, beispielsweise innerhalb eines ZigBee-Netzwerks.

Wenn das Datenmodul 20 nicht als Router verwendet werden soll, wird es bei dem bereits beschriebenen Schritt 209 ausgeschaltet. Abweichend von der Darstellung in Figur 12 kann abhängig von dem Grund des Abschaltens noch eine Meldung an eine das Datenmodul 20 benutzende Person erfolgen, beispielsweise über eine Benutzerschnittstelle 28.

Wenn das Datenmodul 20 als Router verwendet werden soll, wird es bei dem folgenden Schritt **278** als Router verwendet.

Bei einem Schritt **279** wird geprüft, ob das Datenmodul ausgeschaltet werden soll. Dazu wird beispielsweise eine Benutzerschnittstelle 40 abgefragt oder geprüft, ob die Versorgungsspannung U noch einen Mindestwert Uₘᵢₙ aufweist oder überschreitet.

Wenn das Datenmodul nicht ausgeschaltet werden soll, wird es weiterhin als Router verwendet, Schritt 278.

Wenn das Datenmodul ausgeschaltet werden soll, wird es bei dem bereits beschriebenen Schritt 209 ausgeschaltet.

Abweichend von der Darstellung in den Figuren 10 bis 13 kann abhängig von dem Grund des Abschaltens vor dem Schritt 209 des Ausschaltens noch eine Meldung an eine das Datenmodul 20 benutzende Person erfolgen, beispielsweise über eine Benutzerschnittstelle 28.

Der Ablauf von Messung, Prüfung der Erfüllung vorbestimmter Bedingungen, Auswertung des Messergebnisses und Prüfung der Plausibilität kann anders als anhand der Figuren 10 bis 13 dargestellt erfolgen.

Das Anpassen von Parametern wie der Helligkeit einer Lichtquelle, Schwellenwerten und Filterparametern kann einen Betrieb mit geringerer Leistungsaufnahme ermöglichen. Beispielsweise wird zunächst eine geringere Intensität von Beleuchtungslicht verwendet, und nur wenn diese nicht ausreicht, wird die Intensität des Lichts erhöht.

### Bezugszeichenliste:

- **10**: **Tragbare Vorrichtung**
- **20**: **Datenmodul**
- 22: Oberseite des Datenmoduls 20
- 24: Unterseite des Datenmoduls 20
- 28: Display des Datenmoduls 20
- **30**: **Gehäuse** des Datenmoduls 20
- 32: erstes Gehäuseteil des Gehäuses 30
- 34: zweites Gehäuseteil des Gehäuses 30
- 36: Dichteinrichtung zwischen dem ersten Gehäuseteil 32 und dem zweiten Gehäuseteil 34
- 38: äußere Oberfläche des Gehäuses 30
- **40**: **elastischer äußerer Oberflächenbereich,** durch die Dichteinrichtung 36 gebildet
- 42: Betätigungssensor zum Erfassen einer Verformung des elastischen äußeren Oberflächenbereichs
- **50**: **konkaver äußerer Oberflächenbereich** des Gehäuses 30
- 52: Magnet unter dem konkaven äußeren Oberflächenbereich 50
- 54: elektrischer Kontakt, einen Teil des konkaven äußeren Oberflächenbereichs 50 bildend
- **60**: **konvexer äußerer Oberflächenbereich** des Gehäuses 30
- 62: Lichtaustrittsfläche an dem konvexen Oberflächenbereich 60
- 64: Lichteintrittsfläche an dem konvexen Oberflächenbereich 60
- 70: Platine
- 72: Steckkontakt in dem Gehäuse 30
- 74: elektronisches Modul in dem Gehäuse 30
- 76: Leistungsquelle in dem Gehäuse 30
- 80: Prozessor
- 82: Beschleunigungssensor zum Erfassen einer Beschleunigung des Datenmoduls 20
- **100**: **Funktionsmodul**
- 102: Oberseite des Funktionsmoduls 100
- 104: Unterseite des Funktionsmoduls 100
- 106: Rand des Funktionsmoduls 100
- **110**: **Tragstruktur** des Funktionsmoduls 100
- 112: Oberseite der Tragstruktur 110
- 114: Unterseite der Tragstruktur 110
- **120**: **strukturierte Schicht** der Tragstruktur 110
- 122: Zentralbereich der strukturierten Schicht 120
- 124: kammartige Struktur der strukturierten Schicht 120
- 126: gekrümmter Finger der kammartigen Struktur 124
- **130**: **unstrukturierte Schicht** der Tragstruktur 110
- 132: Zwischenschicht zwischen strukturierter Schicht 120 und unstrukturierter Schicht 130
- 134: Klebeschicht
- 136: Befestigungspunkt zur mechanischen Verbindung der strukturierten Schicht 120 mit der Zwischenschicht 132
- **140**: **C-förmiger Schlitz** in der Zwischenschicht 132
- 142: Halbinsel innerhalb der C-förmigen Ausnehmung 140
- 144: Steg, die Halbinsel 142 mit der übrigen Zwischenschicht 132 verbindend
- 146: Temperatursensor auf der Halbinsel 142
- **150**: **konvexer äußerer Oberflächenbereich** des Funktionsmoduls 100
- 152: Magnet unter dem konvexen äußeren Oberflächenbereich 150
- 154: elektrischer Kontakt, einen Teil des konvexen äußeren Oberflächenbereichs 150 bildend
- **160**: **Öffnung** des Funktionsmoduls 100
- **170**: **Band** des Funktionsmoduls 100, zur Befestigung am Handgelenk
- **180**: **Gehäuse** an dem Funktionsmodul 100
- 182: Lichtquelle des Funktionsmoduls 100
- 184: Kamera des Funktionsmoduls 100, zum Erfassen codierter Information
- 186: Drucksensor des Funktionsmoduls 100
- 188: Fluidleitung des Funktionsmoduls 100
- 201: Schritt: Einschalten des Datenmoduls
- 202: Schritt: Initialisieren des Datenmoduls
- 203: Schritt: Prüfen der Versorgungsspannung
- 204: Schritt: Melden einer zu geringen Versorgungsspannung
- 209: Schritt: Ausschalten des Datenmoduls
- 214: Schritt: Prüfen, ob ein Funktionsmodul mit dem Datenmodul verbunden ist
- 215: Schritt: Prüfen ob das Funktionsmodul eine Kamera oder ein Scanner umfasst
- 216: Schritt: Initialisieren der Kamera oder des Scanners
- 217: Schritt: Beleuchten eines Objekts
- 218: Schritt: Erfassen des Objekts mittels der Kamera oder des Scanners
- 219: Schritt: Prüfen des Kontrasts
- 220: Schritt: Vergrößern des Lichtstroms auf das Objekt
- 221: Schritt: Auswerten des erfassten Bilds oder Musters
- 222: Schritt: Prüfen der Plausibilität des Ergebnisses
- 223: Schritt: Ändern eines Parameters
- 224: Schritt: Speicher, Senden oder Anzeigen des Ergebnisses
- 225: Schritt: Prüfen, ob das Datenmodul ausgeschaltet werden soll
- 236: Schritt: Messen
- 237: Schritt: Prüfen, ob da Messergebnis in einem vorbestimmten Bereich liegt
- 238: Schritt: Ändern eines Parameters
- 239: Schritt: Auswerten des Messergebnisses
- 240: Schritt: Prüfen der Plausibilität des Messergebnisses
- 241: Schritt: Ändern eines Parameters
- 242: Schritt: Ändern eines Parameters
- 243: Schritt: Speicher, Senden oder Anzeigen des Ergebnisses
- 244: Schritt: Prüfen, ob das Datenmodul ausgeschaltet werden soll
- 255: Schritt: Melden, dass kein Funktionsmodul angeschlossen ist
- 256: Schritt: Abfragen, ob das Datenmodul allein betrieben werden soll
- 257: Schritt: Messen
- 258: Schritt: Auswerten des Messergebnisses
- 259: Schritt: Prüfen, ob das Messergebnis in einem vorbestimmten Bereich liegt
- 260: Schritt: Ändern eines Parameters
- 261: Schritt: Prüfen der Plausibilität des Messergebnisses
- 262: Schritt: Ändern eines Parameters
- 263: Schritt: Speichern, Senden oder Anzeigen des Messergebnisses
- 277: Schritt: Abfragen, ob das Datenmodul als Router betrieben werden soll
- 278: Schritt: Betreiben des Datenmoduls als Router
- 279: Schritt: Prüfen, ob das Datenmodul ausgeschaltet werden soll

## Patentansprüche

1. ***Tragbares* Datenmodul** (20) zur Befestigung am Körper eines menschlichen oder tierischen Lebewesens und zum Erfassen, Verarbeiten, Speichern oder Senden von Daten, mit:
einer **Schnittstelle** (50, 52, 54) zum mechanischen und elektrischen Verbinden des Datenmoduls (20) mit einem Funktionsmodul (100),
wobei die Schnittstelle **Magneten** (52) zur magnetischen Wechselwirkung mit korrespondierenden Magneten (152) des Funktionsmoduls (100) und **elektrische Kontakte** (54) zur Bildung lösbarer elektrisch leitfähiger Verbindungen mit korrespondierenden elektrischen Kontakten (154) des Funktionsmoduls (100) umfasst,
wobei alle elektrischen Kontakte (54) in **mehreren Gruppen** mit jeweils mehreren elektrischen Kontakten (54) angeordnet sind,
wobei alle Abstände zwischen nächst benachbarten elektrischen Kontakten (54) innerhalb einer Gruppe kleiner sind als alle Abstände zwischen je zwei elektrischen Kontakten (54) aus verschiedenen nächst benachbarten Gruppen,
wobei ein **erster Magnet** (52) an einer ersten Gruppe elektrischer Kontakte (54) angeordnet ist und ein **zweiter Magnet** (52) an einer zweiten Gruppe elektrischer Kontakte (54) angeordnet ist.

2. Tragbares Datenmodul (20) gemäß dem vorangehenden Anspruch, ferner mit:
einem **konvexen äußeren Oberflächenbereich** (60) an derselben Seite des Datenmoduls (20), an der auch die elektrischen Kontakte angeordnet sind, zum Eingreifen in eine Ausnehmung (160) des Funktionsmoduls (100);
einem **Sensor** (64) an dem konvexen Oberflächenbereich (60),
wobei der konvexe Oberflächenbereich (60) **keine 2-zählige Rotationssymmetrie** aufweist.

3. Tragbares Datenmodul (20) gemäß einem der vorangehenden Ansprüche, ferner mit:
einem **Gehäuse** (30), das die äußere Oberfläche (38) des tragbaren Datenmoduls (20) bildet;
einem **Betätigungssensor** (42),
wobei das Gehäuse (30) ein erstes Gehäuseteil (32) und ein zweites Gehäuseteil (34) und eine Dichteinrichtung (36) aus einem elastischen Material zwischen dem ersten Gehäuseteil (32) und dem zweiten Gehäuseteil (34) aufweist,
wobei die Dichteinrichtung (36) einen **elastischen Oberflächenbereich** (40) der äußeren Oberfläche des tragbaren Datenmoduls (20) bildet, dessen manuelle Verformung durch den Betätigungssensor (42) erfasst wird.

4. **Tragbares Funktionsmodul** (100) zur Befestigung am Körper eines menschlichen oder tierischen Lebewesens und zum Erfassen eines Messwerts, mit:
einer **Schnittstelle** (150, 152, 154) zum mechanischen und elektrischen Verbinden des Funktionsmoduls (100) mit einem tragbaren Datenmodul (20),
wobei die Schnittstelle (150 ) **Magneten** (152) zur magnetischen Wechselwirkung mit korrespondierenden Magneten (52) des Datenmoduls (20) und **elektrische Kontakte** (154) zur Bildung lösbarer elektrisch leitfähiger Verbindungen mit korrespondierenden elektrischen Kontakten (54) des Datenmoduls (20) umfasst,
wobei alle elektrischen Kontakte (154) in **mehreren Gruppen** mit jeweils mehreren elektrischen Kontakten (154) angeordnet sind,
wobei alle Abstände zwischen nächst benachbarten elektrischen Kontakten (54) innerhalb einer Gruppe kleiner sind als alle Abstände zwischen je zwei elektrischen Kontakten (154) aus verschiedenen nächst benachbarten Gruppen,
wobei ein **erster Magnet** (152) an einer ersten Gruppe elektrischer Kontakte (154) angeordnet ist und ein **zweiter Magnet** (152) an einer zweiten Gruppe elektrischer Kontakte (154) angeordnet ist.

5. Tragbares Funktionsmodul (100) gemäß dem vorangehenden Anspruch, ferner mit:
einem **konkaven äußeren Oberflächenbereich** (160) an derselben Seite des Funktionsmoduls (100), an der auch die elektrischen Kontakte (154) angeordnet sind, zum Aufnehmen eines konvexen Bereichs (60) des Datenmoduls (20);
wobei der konkave Oberflächenbereich (160) **keine 2-zählige Rotationssymmetrie** aufweist.

6. Tragbares Funktionsmodul (100) gemäß einem der Ansprüche 4 und 5, ferner mit:
einer **Tragstruktur** (110), mit der die Magneten (152) und die elektrischen Kontakte (154) mechanisch starr verbunden sind;
einer **Klebeschicht** (134) an einer von den elektrischen Kontakten (154) abgewandten Seite der Tragstruktur (110),
wobei die **Tragstruktur** (110) eine strukturierte Schicht (120) umfasst,
wobei die **strukturierte Schicht** (120) der Tragstruktur (110) eine **kammartige Struktur** (124) umfasst.

7. Tragbares Funktionsmodul (100) gemäß dem vorangehenden Anspruch, bei dem
die kammartige Struktur (124) mehrere parallele oder im Wesentlichen parallele **bogenförmige finger- oder streifenförmige Strukturen** (126) umfasst.

8. Tragbares Funktionsmodul (100) gemäß einem der Ansprüche 6 und 7, bei dem
die strukturierte Schicht (120) mehrere kammartige Strukturen (124) mit jeweils mehreren parallelen oder im Wesentlichen parallelen **bogenförmigen finger- oder streifenförmige Strukturen** (126) umfasst.

9. Tragbares Funktionsmodul (100) gemäß dem vorangehenden Anspruch, bei dem
die kammartigen Strukturen (124) von einem **streifenförmigen** oder im Wesentlichen streifenförmigen oder im Wesentlichen kreuzförmigen oder knochenförmigen **Zentralbereich** (122) der strukturierten Schicht (120) ausgehen,
die Gruppen elektrischer Kontakte (154) an von einander abgewandten Enden des Zentralbereichs (122) der strukturierten Schicht (120) befestigt sind.

10. Tragbares Funktionsmodul (100) gemäß einem der Ansprüche 4 bis 9, wobei
das Funktionsmodul (100) länglich und tailliert ist.

11. Tragbares Funktionsmodul (100) gemäß einem der Ansprüche 4 bis 10, ferner mit:
einer **Kamera** (184) oder einem **Scanner** zum Erfassen codierter Information;
einer **Befestigungseinrichtung** (170) zum lösbaren mechanischen Befestigen des tragbaren Funktionsmoduls (100) an einer Hand oder einem Handgelenk oder einem Unterarm oder einem Oberarm oder einem Oberkörper oder einem Kopf einer Person oder an einem Tier oder an einer weiteren Vorrichtung.

12. Tragbares Funktionsmodul (100) gemäß einem der Ansprüche 4 bis 14, ferner mit
einem **Feuchtesensor** oder einer Schnittstelle zur Kopplung mit einem Feuchtesensor.

13. Tragbares Datenmodul (20) gemäß einem der Ansprüche 1 bis 3 oder tragbares Funktionsmodul (100) gemäß einem der Ansprüche 4 bis 12, bei dem
eine erste Gruppe elektrischer Kontakte (54, 154) **zwischen** dem ersten Magneten (52, 152) und einem dritten Magneten (52, 152) angeordnet ist und eine zweite Gruppe elektrischer Kontakte (54, 154) **zwischen** dem zweiten Magneten (52, 152) und einem vierten Magneten (52, 152) angeordnet ist.

14. Tragbares Datenmodul (20) oder tragbares Funktionsmodul (100) gemäß dem vorangehenden Anspruch, bei dem
der erste Magnet (52, 152), die elektrischen Kontakte (54, 154) der ersten Gruppe und der dritte Magnet (52, 152) in einer Reihe angeordnet sind,
der zweite Magnet (52, 152), die elektrischen Kontakte (54, 154) der zweiten Gruppe und der vierte Magnet (52, 152) in einer Reihe angeordnet sind.

15. Tragbares Datenmodul (20) oder tragbares Funktionsmodul (100) gemäß dem vorangehenden Anspruch, bei dem
die **Reihe** aus dem ersten Magneten (52, 152), den elektrischen Kontakten (54, 154) der ersten Gruppe und dem dritten Magneten (52, 152) **parallel** zu der **Reihe** aus dem zweiten Magneten (52, 152), den elektrischen Kontakten (54, 154) der zweiten Gruppe und dem vierten Magneten (52, 152) angeordnet ist.

## Claims

1. **Wearable data module** (20) for attachment to the body of a human or animal and for acquiring, processing, storing or transmitting data, comprising:
an **interface** (50, 52, 54) for mechanically and electrically connecting the data module (20) to a functional module (100),
wherein the interface comprises **magnets** (52) for magnetic interaction with corresponding magnets (152) of the functional module (100) and **electrical contacts** (54) for forming releasable electrically conductive connections with corresponding electrical contacts (154) of the functional module (100),
wherein all electrical contacts (54) are arranged in a **plurality of groups,** each comprising several electrical contacts (54),
wherein all distances between next adjacent electrical contacts (54) within a group are smaller than all distances between any two electrical contacts (54) from different next adjacent groups,
wherein a **first magnet** (52) is arranged at a first group of electrical contacts (54) and a **second magnet** (52) is arranged at a second group of electrical contacts (54).

2. Wearable data module (20) according to the preceding claim, further comprising:
a **convex outer surface area** (60) at the same side of the data module (20) as the electrical contacts, for engaging in a recess (160) of the functional module (100);
a **sensor** (64) at the convex surface area (60),
wherein the convex surface area (60) does **not** have **2-fold rotational symmetry.**

3. Wearable data module (20) according to one of the preceding claims, further comprising:
a **housing** (30) forming the outer surface (38) of the wearable data module (20);
an **actuation sensor** (42),
wherein the housing (30) comprises a first housing part (32) and a second housing part (34) and a sealing means (36) made of an elastic material between the first housing part (32) and the second housing part (34),
wherein the sealing means (36) forms an **elastic surface area** (40) of the outer surface of the wearable data module (20), the manual deformation of which is detected by the actuation sensor (42).

4. **Wearable functional module** (100) for attachment to the body of a human or animal and for acquiring a measured value, comprising:
an **interface** (150, 152, 154) for mechanically and electrically connecting the functional module (100) to a wearable data module (20),
wherein the interface (150) comprises **magnets** (152) for magnetic interaction with corresponding magnets (52) of the data module (20) and **electrical contacts** (154) for forming releasable electrically conductive connections with corresponding electrical contacts (54) of the data module (20),
wherein all electrical contacts (154) are arranged in a **plurality of groups,** each comprising a plurality of electrical contacts (154),
wherein all distances between next adjacent electrical contacts (54) within a group are smaller than all distances between any two electrical contacts (154) from different next adjacent groups,
wherein a **first magnet** (152) is arranged at a first group of electrical contacts (154) and a **second magnet** (152) is arranged at a second group of electrical contacts (154).

5. Wearable functional module (100) according to the preceding claim, further comprising:
a **concave outer surface area** (160) at the same side of the functional module (100) as the electrical contacts (154), for receiving a convex region (60) of the data module (20);
wherein the concave surface area (160) does **not** have **2-fold rotational symmetry.**

6. Wearable functional module (100) according to one of the claims 4 and 5, further comprising:
a **support structure** (110) to which the magnets (152) and the electrical contacts (154) are mechanically rigidly connected;
an **adhesive layer** (134) at a side of the support structure (110) facing away from the electrical contacts (154),
wherein the **support structure** (110) comprises a structured layer (120),
wherein the **structured layer** (120) of the support structure (110) comprises a **comb-like structure** (124).

7. Wearable functional module (100) according to the preceding claim,
wherein
the comb-like structure (124) comprises a plurality of parallel or substantially parallel **arcuate finger- or strip-shaped structures** (126).

8. Wearable functional module (100) according to one of the claims 6 and 7,
wherein
the structured layer (120) comprises a plurality of comb-like structures (124), each comprising a plurality of parallel or substantially parallel **arcuate finger- or strip-shaped structures** (126).

9. Wearable functional module (100) according to one of the claims 8 and 9,
wherein
the comb-like structures (124) extend from a **strip-shaped** or substantially strip-shaped or substantially cross-shaped or bone-shaped **central region** (122) of the structured layer (120),
the groups of electrical contacts (154) are attached at ends of the central region (122) of the structured layer (120) that face away from each other (120).

10. Wearable functional module (100) according to one of the claims 4 to 9,
wherein
the functional module (100) is elongated and waisted.

11. Wearable functional module (100) according to one of the claims 4 to 10, further comprising:
a **camera** (184) or a **scanner** for capturing encoded information;
a **fastening device** (170) for releasably mechanically fastening the wearable functional module (100) to a person's hand or wrist or forearm or upper arm or torso or head, or to an animal or to another device.

12. Wearable functional module (100) according to one of the claims 4 to 11, further comprising
a **moisture sensor** or an interface for coupling to a moisture sensor.

13. Wearable data module (20) according to one of the claims 1 to 3 or wearable functional module (100) according to one of the claims 4 to 12, wherein
a first group of electrical contacts (54, 154) is arranged **between** the first magnet (52, 152) and a third magnet (52, 152), and a second group of electrical contacts (54, 154) is arranged **between** the second magnet (52, 152) and a fourth magnet (52, 152).

14. Wearable data module (20) or wearable functional module (100) according to the preceding claim, wherein
the first magnet (52, 152), the electrical contacts (54, 154) of the first group, and the third magnet (52, 152) are arranged in a row,
the second magnet (52, 152), the electrical contacts (54, 154) of the second group, and the fourth magnet (52, 152) are arranged in a row.

15. Wearable data module (20) or wearable functional module (100) according to the preceding claim, wherein
the **row** consisting of the first magnet (52, 152), the electrical contacts (54, 154) of the first group and the third magnet (52, 152) is arranged **parallel** to the **row** consisting of the second magnet (52, 152), the electrical contacts (54, 154) of the second group, and the fourth magnet (52, 152).

## Revendications

1. **Module de données portable** (20) destiné à être fixé au corps d'un être vivant humain ou animal et à acquérir, traiter, stocker ou transmettre des données, comprenant :
une **interface** (50, 52, 54) permettant de connecter mécaniquement et électriquement le module de données (20) à un module fonctionnel (100),
l'interface comprenant des **aimants** (52) destinés à une interaction magnétique avec des aimants correspondants (152) du module fonctionnel (100) et des **contacts électriques** (54) destinés à former des connexions électriquement conductrices pouvant être désolidarisées avec des contacts électriques correspondants (154) du module fonctionnel (100),
tous les contacts électriques (54) étant disposés en **plusieurs groupes** comprenant chacun plusieurs contacts électriques (54),
toutes les distances entre les contacts électriques (54) immédiatement voisins au sein d'un groupe étant inférieures à toutes les distances entre deux contacts électriques (54) appartenant respectivement à des groupes différents immédiatement voisins,
un **premier aimant** (52) étant disposé au niveau d'un premier groupe de contacts électriques (54) et un **deuxième aimant** (52) étant disposé au niveau d'un deuxième groupe de contacts électriques (54).

2. Module de données portable (20) selon la revendication précédente, comprenant en outre :
une zone de **surface extérieure convexe** (60) située du même côté du module de données (20) que celui sur lequel sont disposés les contacts électriques, pour s'engager dans un évidement (160) du module fonctionnel (100) ;
un **capteur** (64) situé au niveau de la zone de surface convexe (60),
la zone de surface convexe (60) **ne** présentant pas de **symétrie de rotation d'ordre 2.**

3. Module de données portable (20) selon l'une des revendications précédentes, comprenant en outre :
un **boîtier** (30) formant la surface extérieure (38) du module de données portable (20) ;
un **capteur d'actionnement** (42),
le boîtier (30) comportant une première partie de boîtier (32) et une deuxième partie de boîtier (34), ainsi qu'un dispositif d'étanchéité (36) en un matériau élastique entre la première partie de boîtier (32) et la deuxième partie de boîtier (34),
le dispositif d'étanchéité (36) formant une **zone de surface élastique** (40) de la surface extérieure du module de données portable (20), dont la déformation manuelle est détectée par le capteur d'actionnement (42).

4. **Module fonctionnel portable** (100) destiné à être fixé au corps d'un être vivant humain ou animal et à acquérir une valeur de mesure, comprenant :
une **interface** (150, 152, 154) permettant de connecter mécaniquement et électriquement le module fonctionnel (100) à un module de données portable (20),
l'interface (150) comprenant des **aimants** (152) destinés à une interaction magnétique avec des aimants correspondants (52) du module de données (20) et des **contacts électriques** (154) destinés à former des connexion électriquement conductrices pouvant être désolidarisées avec des contacts électriques correspondants (54) du module de données (20),
tous les contacts électriques (154) étant disposés en **plusieurs groupes** comprenant chacun plusieurs contacts électriques (154),
toutes les distances entre les contacts électriques (54) immédiatement voisins au sein d'un groupe étant inférieures à toutes les distances entre deux contacts électriques (154) appartenant respectivement à des groupes différents immédiatement voisins,
un **premier aimant** (152) étant disposé au niveau d'un premier groupe de contacts électriques (154) et un **deuxième aimant** (152) étant disposé au niveau d'un deuxième groupe de contacts électriques (154).

5. Module fonctionnel portable (100) selon la revendication précédente, comprenant en outre :
une **zone de surface extérieure concave** (160) située du même côté du module fonctionnel (100) que celui sur lequel sont disposés les contacts électriques (154), pour recevoir une zone convexe (60) du module de données (20) ;
la zone de surface concave (160) **ne** présentant pas de **symétrie de rotation d'ordre 2.**

6. Module fonctionnel portable (100) selon l'une des revendications 4 et 5, comprenant en outre :
une **structure de support** (110) à laquelle les aimants (152) et les contacts électriques (154) sont connectés de manière mécaniquement rigide ;
une **couche adhésive** (134) située sur un côté de la structure de support (110) opposé aux contacts électriques (154),
la **structure de support** (110) comprenant une couche structurée (120),
la **couche structurée** (120) de la structure de support (110) comprenant une **structure en forme de peigne** (124).

7. Module fonctionnel portable (100) selon la revendication précédente, dans lequel
la structure en forme de peigne (124) comprend plusieurs **structures arquées en forme de doigts ou de bandes** (126), parallèles ou sensiblement parallèles.

8. Module fonctionnel portable (100) selon l'une des revendications 6 et 7, dans lequel
la couche structurée (120) comprend plusieurs structures en forme de peigne (124), comprenant chacune plusieurs **structures arquées en forme de doigts ou de bandes** (126), parallèles ou sensiblement parallèles.

9. Module fonctionnel portable (100) selon la revendication précédente, dans lequel
les structures en forme de peigne (124) s'étendent à partir d'une **zone centrale** (122) de la couche structurée (120), en **forme de bande** ou sensiblement en forme de bande ou sensiblement en forme de croix ou en forme d'os,
les groupes de contacts électriques (154) étant fixés aux extrémités de la zone centrale (122) de la couche structurée (120), ces extrémités étant opposées l'une à l'autre.

10. Module fonctionnel portable (100) selon l'une des revendications 4 à 9, dans lequel
le module fonctionnel (100) est allongé et présente un étranglement.

11. Module fonctionnel portable (100) selon l'une des revendications 4 à 10, comprenant en outre :
une **caméra** (184) ou un **scanner** pour acquérir des informations codées ;
un **dispositif de fixation** (170) permettant de fixer mécaniquement, de manière à pouvoir le désolidariser, le module fonctionnel portable (100) à une main ou à un poignet ou à un avant-bras ou à un bras ou à la partie supérieure du corps ou à la tête d'une personne, ou à un animal ou à un autre appareil.

12. Module fonctionnel portable (100) selon l'une des revendications 4 à 14, comprenant en outre
un **capteur d'humidité** ou une interface pour le couplage à un capteur d'humidité.

13. Module de données portable (20) selon l'une des revendications 1 à 3 ou module fonctionnel portable (100) selon l'une des revendications 4 à 12, dans lequel
un premier groupe de contacts électriques (54, 154) est disposé **entre** le premier aimant (52, 152) et un troisième aimant (52, 152), et un deuxième groupe de contacts électriques (54, 154) est disposé **entre** le deuxième aimant (52, 152) et un quatrième aimant (52, 152).

14. Module de données portable (20) ou module fonctionnel portable (100) selon la revendication précédente, dans lequel
le premier aimant (52, 152), les contacts électriques (54, 154) du premier groupe et le troisième aimant (52, 152) sont disposés en une rangée,
le deuxième aimant (52, 152), les contacts électriques (54, 154) du deuxième groupe et le quatrième aimant (52, 152) sont disposés en une rangée.

15. Module de données portable (20) ou module fonctionnel portable (100) selon la revendication précédente, dans lequel
la **rangée** constituée du premier aimant (52, 152), des contacts électriques (54, 154) du premier groupe et du troisième aimant (52, 152) est disposée **parallèlement** à la **rangée** constituée du deuxième aimant (52, 152), des contacts électriques (54, 154) du deuxième groupe et du quatrième aimant (52, 152).
